# EUROPÄISCHE PATENTSCHRIFT

(11) **EP 4 225 163 B1**
(45) Veröffentlichungstag und Bekanntmachung des Hinweises auf die Patenterteilung: **03.12.2025**
(21) Anmeldenummer: 21787469.2
(22) Anmeldetag: 08.10.2021
(51) Int. Cl.: A61B 17/128, A61B 17/122

(54) **APPLIKATIONSSYSTEM FÜR MEDIZINISCHE CLIPS**
APPLICATION SYSTEM FOR MEDICAL CLIPS
SYSTÈME D'APPLICATION POUR AGRAFES MÉDICALES

(30) Priorität: 09.10.2020 DE 102020126581
(43) Veröffentlichungstag der Anmeldung: 16.08.2023
(73) Patentinhaber: Aesculap AG, 78532 Tuttlingen (DE)
(72) Erfinder: PLEIL, Thomas, 78073 Bad Dürrheim (DE); SCHOLTEN, Thomas, 78532 Tuttlingen (DE); WILLMANN, Michael, 78048 Villingen-Schwenningen (DE); HAPPLE, Alexander, 78183 Hüfingen (DE); BEGER, Jens, 78532 Tuttlingen (DE)
(74) Vertreter: Hoeger, Stellrecht & Partner Patentanwälte mbB
(86) Internationale Anmeldenummer: PCT/EP2021/077858
(87) Internationale Veröffentlichungsnummer: WO 2022/074191

(56) Entgegenhaltungen:
- DE-U1- 202010 008 941
- JP-A- 2006 081 597
- US-A1- 2018 271 527
- US-A1- 2018 271 534

## Beschreibung

Die vorliegende Erfindung betrifft ein Applikationssystem für medizinische Clips, umfassend mindestens ein erstes Applikationsinstrument zum Anlegen eines ersten Typs eines medizinischen Clips und mindestens einen ersten medizinischen Clip vom ersten Typ, wobei das erste Applikationsinstrument zwei relativ zueinander bewegbare erste Werkzeugelemente umfasst, wobei der mindestens eine erste medizinische Clip vom ersten Typ einen ersten proximalen Endbereich und zwei erste Verbindungsabschnitte aufweist, welche sich distalseitig an den ersten, einen ersten Volumenbereich definierenden proximalen Endbereich anschließen und den ersten proximalen Endbereich mit zwei relativ zueinander bewegbaren Klemmarmen des mindestens einen ersten Clips vom ersten Typ verbinden, wobei die zwei ersten Werkzeugelemente zwischen sich eine in distaler Richtung geöffnete erste Endbereichsaufnahme zum formschlüssigen oder im Wesentlichen formschlüssigen Aufnehmen des ersten proximalen Endbereichs und jeweils eine erste Verbindungsabschnittsaufnahme zum mindestens teilweisen Aufnehmen jeweils eines ersten Aufnahmeabschnitts der ersten Verbindungsabschnitte umfassen, wobei an mindestens einem der zwei ersten Werkzeugelemente mindestens ein erstes Unterscheidungselement derart angeordnet oder ausgebildet ist, dass es in einen zweiten Volumenbereich eingreift, welcher von einem zweiten proximalen Endbereich eines zweiten medizinischen Clips von einem zweiten Typ definiert wird, wobei sich vom zweiten proximalen Endbereich in distaler Richtung zwei zweite, jeweils einen zweiten Aufnahmeabschnitt umfassende Verbindungsabschnitte weg erstrecken, und wobei der erste Volumenbereich und der zweite Volumenbereich nur teilweise überlappen unter der Zwangsbedingung, dass die zweiten Aufnahmeabschnitte in jeweils einer ersten Verbindungsabschnittsaufnahme der zwei ersten Werkzeugelemente aufgenommen sind.

Applikationssysteme der eingangs beschriebenen Art werden insbesondere zu neurochirurgischen Behandlung von kranialen Aneurysmen eingesetzt. Sie dienen der Applikation von Aneurysmenclips, die beispielsweise aus Titan oder Phynox ausgebildet sind.

Ein Problem bei derartigen Applikationssystemen ist insbesondere, dass mit mit ihnen dem Grunde nach unterschiedliche Cliptypen appliziert werden können. Dies ist jedoch insbesondere dann problematisch, wenn sich die Cliptypen unterscheiden, beispielsweise in Größe, Form und/oder Schließkraft, und der Einsatz des bekannten ersten Applikationsinstruments zu einer Beschädigung bestimmter Cliptypen führen kann.

Aus der JP 2006 081597 A sind Applikatoren für Aneurysmenclips bekannt. Die DE 20 2010 008 941 U1 betrifft ein chirurgisches Instrument. Ein Clipanlegeinstrument mit Stabilisierungselement ist in der US 2018/271527 A1 beschrieben. Ein weiteres Clipapplikationsinstrument mit einem Stabilisierungsglied ist aus der US 2018/271534 bekannt.

Es ist daher eine Aufgabe der vorliegenden Erfindung, ein Applikationssystem für medizinische Clips der eingangs beschriebenen Art so zu verbessern, dass medizinische Clips insbesondere sicher und zuverlässig appliziert werden können.

Diese Aufgabe wird bei einem Applikationssystem für medizinische Clips der eingangs beschriebenen Art erfindungsgemäß dadurch gelöst, dass das Applikationssystem mindestens ein zweites Applikationsinstrument zum Anlegen eines zweiten Typs eines medizinischen Clips und mindestens einen zweiten medizinischen Clip vom zweiten Typ umfasst, welcher zwei relativ zueinander bewegbare zweite Klemmarme, die sich distalseitig an die zwei zweiten Verbindungsabschnitte anschließen, umfasst, dass das mindestens eine zweite Applikationsinstrument zwei relativ zueinander bewegbare zweite Werkzeugelemente umfasst, dass die zwei zweiten Werkzeugelemente zwischen sich eine in distaler Richtung geöffnete zweite Endbereichsaufnahme zum formschlüssigen oder im Wesentlichen formschlüssigen Aufnehmen des zweiten proximalen Endbereichs und jeweils eine zweite Verbindungsabschnittsaufnahme zum mindestens teilweisen Aufnehmen jeweils eines der zweiten Aufnahmeabschnitte umfassen, dass an mindestens einem der zwei zweiten Werkzeugelemente mindestens ein zweites Unterscheidungselement derart angeordnet oder ausgebildet ist, dass es in den ersten Volumenbereich eingreift unter der Zwangsbedingung, dass die ersten Aufnahmeabschnitte in jeweils einer zweiten Verbindungsabschnittsaufnahme der zwei zweiten Werkzeugelemente aufgenommen sind.

Die vorgeschlagene Weiterbildung eines Applikationssystems für medizinische Clips der eingangs beschriebenen Art ermöglicht es insbesondere, zu verhindern, dass der zweite proximale Endbereich des zweiten medizinischen Clips vom zweiten Typ aufgenommen werden kann unter der angegebenen Zwangsbedingung, also dass die zweiten Aufnahmeabschnitte in jeweils einer der beiden ersten Verbindungsabschnittsaufnahmen der zwei ersten Werkzeugelemente aufgenommen sind. Da der zweite proximale Endbereich des zweiten Clips vom zweiten Typ nicht in der ersten Endbereichsaufnahme unter der Zwangsbedingung aufgenommen werden kann, kann er auch nicht, insbesondere nicht unabsichtlich, appliziert werden. Die vorgeschlagene Weiterbildung ermöglicht insbesondere eine zuverlässige mechanische und damit automatische Unterscheidung unterschiedlicher Cliptypen mit dem ersten Applikationsinstrument. Das mindestens eine erste Unterscheidungselement verhindert dabei insbesondere, dass der zweite proximale Endbereich des zweiten medizinischen Clips vom zweiten Typ in der ersten Endbereichsaufnahme aufnehmbar ist. Anders als beispielsweise bei einer Kodierung von Applikationsinstrumenten und Clips mit Farben, können so insbesondere keine Verwechslungen passieren, da es das mindestens eine erste Unterscheidungselement verhindert, dass mit dem ersten Applikationsinstrument ein medizinischer Clip, der nicht vom ersten Typ ist, aufgenommen werden kann. Insbesondere kann das mindestens eine erste Unterscheidungselement derart angeordnet oder ausgebildet sein, dass beim Bewegen der ersten Werkzeugelemente aufeinander zu der Clip, der nicht vom ersten Typ ist, aus der ersten Endbereichsaufnahme herausgedrückt wird oder herausspringt. So kann ein Operateur sofort erkennen, dass versucht wurde, den falschen Cliptyp zu applizieren. Das wie vorgeschlagen ausgebildete Applikationssystem ermöglicht es insbesondere, technische Designmerkmale unterschiedlicher medizinischer Clips, beispielsweise unterschiedlicher Generationen von Clips, zu nutzen, um eine definierte Zuordnung zu korrespondierenden Applikationsinstrumenten zu ermöglichen, wodurch ein Verwechslungsrisiko für die Applikation unterschiedlicher medizinischer Clips, also insbesondere unterschiedlicher Typen, minimiert wird. Insbesondere können derartige Designunterschiede im proximalen Endbereich der unterschiedlichen Cliptypen realisiert werden. Das mindestens eine erste Unterscheidungselement kann insbesondere den Einsatz des ersten Applikationsinstruments mit medizinischen Clips, die nicht vom ersten Typ sind, erschweren oder gar unmöglich machen. Gemäß der Erfindung ist vorgesehen, dass das Applikationssystem mindestens ein zweites Applikationsinstrument zum Anlegen eines zweiten Typs eines medizinischen Clips und mindestens einen zweiten medizinischen Clip vom zweiten Typ umfasst, welcher zwei relativ zueinander bewegbare zweite Klemmarme, die sich distalseitig an die zwei zweiten Verbindungsabschnitte anschließen, umfasst, dass das mindestens eine zweite Applikationsinstrument zwei relativ zueinander bewegbare zweite Werkzeugelemente umfasst, dass die zwei zweiten Werkzeugelemente zwischen sich eine in distaler Richtung geöffnete zweite Endbereichsaufnahme zum formschlüssigen oder im Wesentlichen formschlüssigen Aufnehmen des zweiten proximalen Endbereichs und jeweils eine zweite Verbindungsabschnittsaufnahme zum mindestens teilweisen Aufnehmen jeweils eines der zweiten Aufnahmeabschnitte umfassen, dass an mindestens einem der zwei zweiten Werkzeugelemente mindestens ein zweites Unterscheidungselement derart angeordnet oder ausgebildet ist, dass es in den ersten Volumenbereich eingreift unter der Zwangsbedingung, dass die ersten Aufnahmeabschnitte in jeweils einer zweiten Verbindungsabschnittsaufnahme der zwei zweiten Werkzeugelemente aufgenommen sind. Das mindestens eine zweite Applikationsinstrument ist also insbesondere in analoger Weise wie das erste Applikationsinstrument ausschließlich zum Aufnehmen und Applizieren eines zweiten medizinischen Clips vom zweiten Typ geeignet. Medizinische Clips vom ersten Typ können mit ihrem ersten proximalen Endbereich nicht oder nur sehr eingeschränkt in der zweiten Endbereichsaufnahme aufgenommen werden. Vorzugsweise ist das mindestens eine zweite Unterscheidungselement derart angeordnet, dass der mindestens eine erste medizinische Clip vom ersten Typ aus der zweiten Endbereichsaufnahme des zweiten Applikationsinstruments herausspringt, wenn er mit dem zweiten Applikationsinstrument gefasst wird. Mit einem solchen System lassen sich mindestens zwei unterschiedliche Cliptypen mit den zu diesen korrespondierenden mindestens zwei Applikationsinstrumenten sicher unterscheiden und in definierter und zuverlässiger Weise applizieren. So können insbesondere Verwechslungen in einem Operationssaal, wo häufig nur eingeschränkte Sichtbedingungen herrschen, zusätzlich zu optischen Unterscheidungsmerkmalen, die optional vorgesehen sein können oder sich aufgrund einer unterschiedlichen Bauform der Cliptypen ergeben, eine zuverlässige mechanische Unterscheidung der Cliptypen mit den beiden Applikationsinstrumenten realisiert werden. Die Unterscheidungselemente können insbesondere in Form von Vorsprüngen, Rücksprüngen oder speziell geformten Anlageflächen ausgebildet sein, die mit bestimmten Formen der Cliptypen, beispielsweise Formen von Endbereichen derselben, mechanisch zusammenwirken und entweder das vollständige Aufnehmen des medizinischen Clips eines bestimmten Typs mit dem zugeordneten Applikationsinstrument ermöglichen, um den Clip applizieren zu können, oder eben nicht.

Vorteilhaft ist es, wenn das mindestens eine zweite Applikationsinstrument eine zweite Betätigungseinrichtung umfasst zum Bewegen, insbesondere zum Verschwenken, der zwei zweiten Werkzeugelemente relativ zueinander von einer zweiten Aufnahmestellung, in welcher ein zum zweiten Applikationsinstrument korrespondierender zweiter medizinischer Clip vom zweiten Typ mit seinem zweiten Endbereich in der zweiten Endbereichsaufnahme aufnehmbar ist, in eine zweite Applikationsstellung, in welcher die zwei zweiten Werkzeugelemente aufeinander zu und die zweiten Klemmarme voneinander weg bewegt sind zum Applizieren des zweiten Clips vom zweiten Typ. Ein derart ausgebildetes Applikationsinstrument ermöglicht insbesondere eine einfache und zuverlässige Handhabung zweiter medizinischer Clips vom zweiten Typ, um diese in gewünschter Weise zuverlässig applizieren zu können.

Um zweite medizinische Clips vom zweiten Typ sicher handhaben zu können, also insbesondere derart, dass sie nicht unbeabsichtigt vom zweiten Applikationsinstrument abfallen können, ist es vorteilhaft, wenn die zweiten Werkzeugelemente des zweiten Applikationsinstruments den zweiten proximalen Endbereich in der zweiten Aufnahmestellung und in der zweiten Applikationsstellung teilweise umgreifen.

Günstigerweise sind die zwei zweiten Werkzeugelemente des mindestens einen zweiten Applikationsinstruments relativ zueinander bewegbar sind, insbesondere um eine gemeinsame zweite Schwenkachse relativ zueinander verschwenkbar. Auf diese Weise lassen sich beliebige und insbesondere einfach und zuverlässig handzuhabende zweite Applikationsinstrumente ausbilden. Insbesondere relativ zueinander verschwenkbare zweite Werkzeugelement lassen sich gefühlvoll in definierter Weise relativ zueinander bewegen.

Gemäß einer weiteren bevorzugten Ausführungsform der Erfindung kann vorgesehen sein, dass das mindestens eine erste Applikationsinstrument eine erste Betätigungseinrichtung umfasst zum Bewegen, insbesondere zum Verschwenken, der zwei ersten Werkzeugelemente relativ zueinander von einer ersten Aufnahmestellung, in welcher ein zum ersten Applikationsinstrument korrespondierender erster medizinischer Clip vom ersten Typ mit seinem ersten Endbereich in der ersten Endbereichsaufnahme aufnehmbar ist, in eine erste Applikationsstellung, in welcher die zwei ersten Werkzeugelemente aufeinander zu und die ersten Klemmarme voneinander weg bewegt sind zum Applizieren des ersten Clips vom ersten Typ. Mit einem derart ausgebildeten ersten Applikationsinstrument lassen sich medizinische Clips vom ersten Typ einfach und sicher handhaben und in definierter und gewünschter Weise applizieren.

Um ein unbeabsichtigtes Lösen medizinischer Clips vom ersten Typ vom ersten Applikationsinstrument möglichst zu verhindern, ist es vorteilhaft, wenn die ersten Werkzeugelemente des ersten Applikationsinstruments den ersten proximalen Endbereich in der ersten Aufnahmestellung und in der ersten Applikationsstellung teilweise umgreifen.

Günstig ist es, wenn die zwei ersten Werkzeugelemente des mindestens einen ersten Applikationsinstruments relativ zueinander bewegbar sind. Insbesondere ist es vorteilhaft, wenn sie um eine gemeinsame erste Schwenkachse relativ zueinander verschwenkbar sind. Derart ausgebildete erste Werkzeugelemente ermöglichen es insbesondere, einen proximalen Endbereich eines ersten medizinischen Clips vom ersten Typ aufzunehmen und durch eine Relativbewegung der zwei ersten Werkzeugelemente zueinander, beispielsweise eine Schwenkbewegung, zu applizieren.

Günstig ist es, wenn sich die ersten Aufnahmeabschnitte direkt oder im Wesentlichen direkt an den ersten proximalen Endbereich anschließen und/oder wenn sich die zweiten Aufnahmeabschnitte direkt oder im Wesentlichen direkt an den zweiten proximalen Endbereich. Medizinische Clips in dieser Weise auszubilden ermöglicht es insbesondere, Endbereiche der medizinischen Clips in der jeweiligen Endbereichsaufnahme aufzunehmen und mit in distaler Richtung weisenden Bereichen der Werkzeugelemente der jeweiligen Applikationsinstrumente, die jeweils eine Verbindungsabschnittsaufnahme aufweisen, an den Clips an den jeweiligen Aufnahmeabschnitten anzugreifen, um durch Bewegen der Werkzeugelemente relativ zueinander die mit dem jeweiligen Applikationsinstrument aufgenommenen Clips zu öffnen und wieder zu schließen.

Günstigerweise umfasst der erste proximale Endbereich ein erstes vorspannendes Element und/oder der zweite proximale Endbereich ein zweites vorspannendes Element. Der proximale Endbereich dient somit insbesondere nicht nur zur Unterscheidung medizinischer Clips unterschiedlicher Typen, sondern insbesondere auch dazu, die Klemmarme gegeneinander vorzuspannen. Insbesondere können die Klemmarme der medizinischen Clips entgegen der Wirkung der vorspannenden Elemente voneinander weg bewegt werden. Die hierzu erforderliche Öffnungskraft kann beispielsweise über die Verbindungsabschnittsaufnahmen der Werkzeugelemente der Applikationsinstrumente auf die Aufnahmeabschnitte der medizinischen Clips eingeleitet werden.

Vorteilhaft ist es, wenn die ersten Klemmarme in einer ersten Grundstellung des mindestens einen ersten Clips vom ersten Typ aneinander anliegen und entgegen der Wirkung des ersten vorspannenden Elements von der ersten Grundstellung in eine erste Öffnungsstellung voneinander weg bewegbar sind und/oder wenn die zweiten Klemmarme in einer zweiten Grundstellung des mindestens einen zweiten Clips vom zweiten Typ aneinander anliegen und entgegen der Wirkung des zweiten vorspannenden Elements von der zweiten Grundstellung in eine zweite Öffnungsstellung voneinander weg bewegbar sind. Wie bereits erläutert lassen sich so die Clips mit einem zu diesen korrespondierend ausgebildeten Applikationsinstrument in definierter Weise öffnen und auch wieder schließen. Das vorspannende Element kann insbesondere auch eine sichere Positionierung des jeweiligen Clips zwischen den zugeordneten Werkzeugelementen vorgeben. Insbesondere kann ein Clip so unabhängig davon, ob er die Grundstellung oder eine Öffnungsstellung einnimmt, stets klemmend am Applikationsinstrument gehalten werden.

Gemäß einer weiteren bevorzugten Ausführungsform der Erfindung kann vorgesehen sein, dass sich die ersten Verbindungsabschnitte in einem ersten Schlussbereich des mindestens einen ersten Clips vom ersten Typ kreuzen und/oder dass sich die zweiten Verbindungsabschnitte in einem zweiten Schlussbereich des mindestens einen zweiten Clips vom zweiten Typ kreuzen. Eine derartige Ausgestaltung ermöglicht es insbesondere, dass die Klemmarme der jeweiligen Clips durch Bewegen der Aufnahmeabschnitte aufeinander zu, wenn diese proximalseitig des Schlussbereichs angeordnet oder ausgebildet sind, voneinander weg bewegt werden können. Zum Applizieren der Clips können dann die Werkzeugelemente des jeweiligen Applikationsinstruments aufeinander zu bewegt werden. Dadurch wird der Clip geöffnet. Zum Applizieren werden die Werkzeugelemente wieder voneinander weg bewegt. Insbesondere das vorspannende Element führt dann dazu, dass die Klemmarme wieder aufeinander zu bewegt werden, insbesondere so weit, dass sie gegebenenfalls aneinander anliegen oder zwischen sich ein Hohlorgan, beispielsweise ein Blutgefäß oder eine Aussackung desselben, einklemmen können.

Vorzugsweise ist der erste Schlussbereich distalseitig der ersten Aufnahmeabschnitte angeordnet oder ausgebildet und/oder ist der zweite Schlussbereich distalseitig der zweiten Aufnahmeabschnitte angeordnet oder ausgebildet. Wie bereits erläutert können so die Klemmarme voneinander weg bewegt werden, indem die jeweiligen Aufnahmeabschnitte aufeinander zu bewegt werden, beispielsweise durch Bewegen der zusammenwirkenden Werkzeugelemente des jeweiligen Applikationsinstruments aufeinander zu.

Günstigerweise sind der erste Schlussbereich und/oder der zweite Schlussbereich in Form eines Durchsteckschlusses ausgebildet. Clips mit Durchsteckschlüssen haben insbesondere den Vorteil, dass die Verbindungsabschnitte aneinander geführt werden. So können unerwünschte Torsionen im Bereich der Klemmarme vermieden oder weitgehend vermieden werden. Dies ist insbesondere vorteilhaft, da so das sogenannte "scissoring" vermieden werden kann. Mit anderen Worten kann das gegenseitige Abscheren der Klemmarme beim Schließen des Clips verhindert werden, wodurch zwischen den Klemmarmen eingeklemmtes Gewebe verletzt werden kann.

Auf einfache Weise lassen sich die Clips ausbilden, wenn das erste vorspannende Element in Form einer ersten Schraubenfeder mit mindestens einer vollständigen Windung ausgebildet ist und/oder wenn das zweite vorspannende Element in Form einer zweiten Schraubenfeder mit mindestens einer vollständigen Windung ausgebildet ist. Durch Wahl des Werkstoffs, aus dem die Schraubenfeder ausgebildet ist, sowie deren Größe, können Schließkräfte der Clips in gewünschter Weise vorgegeben werden. Ferner kann die Formgebung der jeweiligen Schraubenfeder dazu genutzt werden, Clips unterschiedlicher Typen mit den Applikationsinstrumenten des Applikationssystems voneinander zu unterscheiden.

Einfach und kostengünstig lassen sich die Clips ausbilden, wenn das erste vorspannende Element und/oder das zweite vorspannende Elemente durch einen Kaltumformprozess ausgebildet sind. Beispielsweise können die vorspannenden Elemente in Form von Schraubenfedern durch Wickeln ausgebildet werden.

Gemäß einer weiteren bevorzugten Ausführungsform der Erfindung kann vorgesehen sein, dass der mindestens eine erste medizinische Clip vom ersten Typ eine erste Clipebene definiert, dass die erste Schraubenfeder eine erste Schraubenfederlängsachse definiert, dass die erste Schraubenfederlängsachse senkrecht zu einer ersten Schraubenfederebene verläuft und dass die erste Schraubenfederebene relativ zur ersten Clipebene um einen ersten Neigungswinkel geneigt ist und/oder dass der mindestens eine zweite medizinische Clip vom zweiten Typ eine zweite Clipebene definiert, dass die zweite Schraubenfeder eine zweite Schraubenfederlängsachse definiert, dass die zweite Schraubenfederlängsachse senkrecht zu einer zweiten Schraubenfederebene verläuft und dass die zweite Schraubenfederebene relativ zur zweiten Clipebene um einen zweiten Neigungswinkel geneigt ist. Die medizinischen Clips vom ersten Typ und vom zweiten Typ können so insbesondere unterschieden werden durch unterschiedliche Neigungswinkel der jeweiligen Schraubenfederebene relativ zur jeweiligen Clipebene. Die Clipebene kann insbesondere definiert werden durch eine von den Aufnahmeabschnitten der Clips aufgespannte Ebene. Insbesondere können die Clips derart ausgebildet sein, dass sich die Aufnahmeabschnitte beim Öffnen und Schließen der Clips in der Clipebene bewegen. Beispielsweise können sich die Klemmarme in der Clipebene erstrecken oder beispielsweise bei bestimmten Ausführungsformen auch aus der Clipebene heraus gekrümmt sein.

Günstig ist es, wenn die erste Schraubenfeder im Uhrzeigersinn gewickelt ist und wenn der erste Neigungswinkel einen positiven Wert aufweist und/oder wenn die zweite Schraubenfeder im Gegenuhrzeigersinn gewickelt ist und wenn der zweite Neigungswinkel einen negativen Wert aufweist. Diese Weiterbildung ermöglicht es insbesondere, Clips unterschiedlicher Typen auf einfache Weise zu unterscheiden. Dies kann insbesondere aufgrund der Neigungswinkel erreicht werden. Diese können durch unterschiedliche Wicklungsrichtungen, also einmal im Uhrzeigersinn und einmal im Gegenuhrzeigersinn bezogen auf die Schraubenfederlängsachse auf einfache Weise realisiert werden. Aufgrund der unterschiedlichen Neigungswinkel definiert die Schraubenfeder, die letztlich mindestens einen Teil des Endbereichs des jeweiligen Clips bildet, einen Volumenbereich. Volumenbereiche mit unterschiedlichen Neigungswinkeln überlappen sich jedoch nicht, so dass durch entsprechende Unterscheidungselemente an den jeweiligen Applikationsinstrumenten die Clips unterschiedlicher Typen mechanisch auf einfache und sichere Weise unterscheidbar sind.

Eine sichere Unterscheidung unterschiedlicher Cliptypen kann insbesondere dadurch sichergestellt werden, dass der erste Neigungswinkel einen Wert in einem Bereich zwischen 0° und etwa 40° aufweist, insbesondere einen Wert in einem Bereich zwischen 0° und etwa 20°, und/oder wenn der zweite Neigungswinkel einen Wert in einem Bereich zwischen 0° und etwa -40° aufweist, insbesondere einen Wert in einem Bereich zwischen 0° und etwa -20°. Neigungswinkel mit positiven und negativen Werten definieren somit Neigungen der Schraubenfederebenen relativ zur jeweiligen Clipebene in unterschiedlichen Richtungen.

Vorteilhaft ist es, wenn dass das mindestens eine erste Applikationsinstrument korrespondierend zu einem ersten medizinischen Clip vom ersten Typ mit einer im Uhrzeigersinn gewickelten ersten Schraubenfeder ausgebildet ist. Auf diese Weise kann eine eindeutige Zuordnung zwischen medizinischen Clips vom ersten Typ und dem zugeordneten ersten Applikationsinstrument erreicht werden.

Vorzugsweise ist das mindestens eine zweite Applikationsinstrument korrespondierend zu einem zweiten medizinischen Clip vom zweiten Typ mit einer im Gegenuhrzeigersinn gewickelten zweiten Schraubenfeder ausgebildet. Mit einem korrespondierenden zweiten Applikationsinstrument können so einfach und zuverlässig zweite medizinische Clips vom zweiten Typ gehandhabt und insbesondere appliziert werden.

Gemäß einer weiteren bevorzugten Ausführungsform der Erfindung kann vorgesehen sein, dass das mindestens eine erste Unterscheidungselement in Form eines mit einem beliebigen medizinischen Clip zusammenwirkendes erstes Kodierelement umfasst und dass in der ersten Endbereichsaufnahme des ersten Applikationsinstruments ausschließlich der zum ersten Kodierelement korrespondierende erste medizinische Clip vom ersten Typ formschlüssig oder im Wesentlichen formschlüssig aufnehmbar ist. Insbesondere kann der proximale Endbereich des ersten medizinischen Clips vom ersten Typ in der ersten Endbereichsaufnahme aufgenommen werden, da das erste Kodierelement nicht in den vom ersten Endbereich definierten ersten Volumenbereich eingreift. Dagegen ist es nicht möglich, einen zweiten medizinischen Clip vom zweiten Typ in die erste Endbereichsaufnahme einzusetzen unter der Zwangsbedingung, dass die Aufnahmeabschnitte in den Verbindungsabschnittsaufnahmen des ersten Applikationsinstruments aufgenommen sind. In diesem Fall ragt das erste Kodierelement in den zweiten Volumenbereich hinein, der vom zweiten Endbereich des zweiten medizinischen Clips vom zweiten Typ definiert wird. Die Folge hiervon ist, dass der zweite Endbereich nicht in die erste Endbereichsaufnahme aufgenommen werden kann.

Um eine definierte Positionierung des ersten Endbereichs in der ersten Endbereichsaufnahme zu ermöglichen, ist es günstig, wenn das mindestens eine erste Kodierelement in Form eines ersten Kodiervorsprungs ausgebildet ist, welcher die erste Endbereichsaufnahme mindestens abschnittsweise seitlich begrenzt. So kann insbesondere ein Verkippen des Clips in der ersten Endbereichsaufnahme verhindert werden.

Vorteilhaft ist es, wenn das erste Applikationsinstrument zwei mit einem beliebigen medizinischen Clip zusammenwirkende erste Kodierelemente umfasst und dass die ersten Kodierelemente seitliche Anlageflächen für voneinander weg weisende Seiten ausschließlich für den in der ersten Endbereichsaufnahme aufgenommenen ersten Endbereich bilden. Die seitlichen Anlageflächen der ersten Kodierelemente können so insbesondere verhindern, dass sich der erste medizinische Clip vom ersten Typ um eine in der ersten Clipebene liegende Achse verdrehen kann, wenn er in der ersten Endbereichsaufnahme aufgenommen ist. Dies ermöglicht eine zuverlässige Applikation erster medizinischer Clips vom ersten Typ mit dem ersten Applikationsinstrument.

Ferner kann es vorteilhaft sein, wenn das mindestens eine zweite Unterscheidungselement in Form eines mit einem beliebigen medizinischen Clip zusammenwirkendes zweites Kodierelement umfasst und wenn in der zweiten Endbereichsaufnahme des zweiten Applikationsinstruments ausschließlich der zum zweiten Kodierelement korrespondierende zweite medizinische Clip vom zweiten Typ formschlüssig oder im Wesentlichen formschlüssig aufnehmbar ist. Das zweite Kodierelement ermöglicht somit insbesondere die Unterscheidung zwischen zweiten medizinischen Clips vom zweiten Typ und beliebigen anderen Clips. So kann insbesondere auf einfache Weise eine mechanische Zuordnung des zweiten Applikationsinstruments und zweiter medizinischer Clips vom zweiten Typ erreicht werden.

Vorzugsweise ist das mindestens eine zweite Kodierelement in Form eines zweiten Kodiervorsprungs ausgebildet, welcher die zweite Endbereichsaufnahme mindestens abschnittsweise seitlich begrenzt. Am zweiten Kodiervorsprung kann sich beispielsweise ein zweiter Endbereich eines zweiten Clips vom zweiten Typ abstützen, wenn der zweite Endbereich in der zweiten Endbereichsaufnahme des zweiten Applikationsinstruments aufgenommen ist.

Günstig ist es, wenn das zweite Applikationsinstrument zwei mit einem beliebigen medizinischen Clip zusammenwirkende zweite Kodierelemente umfasst und wenn die zweiten Kodierelemente seitliche Anlageflächen für voneinander weg weisende Seiten ausschließlich für den in der zweiten Endbereichsaufnahme aufgenommenen zweiten Endbereich bilden. Auf diese Weise kann insbesondere erreicht werden, dass sich der Clip nicht um eine Achse, die in der zweiten Clipebene liegt, verdrehen kann, wenn der zweite Endbereich in der zweiten Endbereichsaufnahme des zweiten Applikationsinstruments aufgenommen ist.

Vorteilhaft ist es, wenn an jedem ersten Werkzeugelemente des ersten Applikationsinstruments jeweils eines der beiden ersten Kodierelemente angeordnet oder ausgebildet ist und/oder wenn an jedem zweiten Werkzeugelement des zweiten Applikationsinstruments jeweils eines der beiden zweiten Kodierelemente angeordnet oder ausgebildet ist. So können die zu den jeweiligen Werkzeugelementen korrespondierenden Clips bereits beim Aufnehmen ihrer proximalen Endbereiche in die jeweilige Endbereichsaufnahme in definierter Weise geführt werden. Wird beispielsweise versucht, einen nicht zum jeweiligen Applikationsinstrument korrespondierenden Clip mit seinem proximalen Endbereich in der Endbereichsaufnahme des jeweiligen Applikationsinstruments aufzunehmen, treten die Kodierelemente mit dem jeweiligen Endbereich des nicht passenden Clips in Wechselwirkung und führen dazu, dass der Clip verdreht wird und aus der jeweiligen Endbereichsaufnahme herausfällt beziehungsweise nicht in dieser aufgenommen werden kann.

Vorteilhaft ist es, wenn das mindestens eine erste Kodierelement am einen der zwei ersten Werkzeugelemente in distaler Richtung über ein proximales Ende des die erste Endbereichsaufnahme begrenzenden anderen ersten Werkzeugelements vorstehend ausgebildet ist und/oder wenn das mindestens eine zweite Kodierelement am einen der zwei zweiten Werkzeugelemente in distaler Richtung über ein proximales Ende des die zweite Endbereichsaufnahme begrenzenden anderen zweiten Werkzeugelements vorstehend ausgebildet ist. Eine solche Ausgestaltung ermöglicht es insbesondere, bezogen auf die jeweilige Clipebene geometrische Unterschiede mechanisch zu detektieren. Beispielsweise abhängig davon, ob der Endbereich eine in die eine oder in die andere Richtung geneigte Schraubenfeder aufweist, ermöglichen oder behindern die jeweiligen Kodierelemente das Aufnehmen eines Endbereichs eines medizinischen Clips in der Endbereichsaufnahme.

Gemäß einer weiteren bevorzugten Ausführungsform der Erfindung kann vorgesehen sein, dass die zwei ersten Werkzeugelemente im Bereich der ersten Endbereichsaufnahme durch Drehung um 180° bezogen auf eine erste Längsachse der ersten Endbereichsaufnahme ineinander überführbar sind und/oder wenn die zwei zweiten Werkzeugelemente im Bereich der zweiten Endbereichsaufnahme durch Drehung um 180° bezogen auf eine zweite Längsachse der zweiten Endbereichsaufnahme ineinander überführbar sind. Diese Ausgestaltung vereinfacht insbesondere eine Konstruktion der jeweiligen Applikationsinstrumente. Beispielsweise ist es so möglich, zwei identische Werkzeugelemente zur Ausbildung der jeweiligen Applikationsinstrumente zu nutzen.

Zur Behandlung von Aneurysmen ist es insbesondere vorteilhaft, wenn der mindestens eine erste medizinische Clip vom ersten Typ und/oder wenn der mindestens eine zweite medizinische Clip vom zweiten Typ in Form von Aneurysmenclips ausgebildet sind.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtansicht eines ersten Ausführungsbeispiels eines ersten Applikationsinstruments mit einem ersten medizinischen Clip vom ersten Typ;
- Figur 2:: eine vergrößerte perspektivische Teilansicht eines distalen Endbereichs des ersten Applikationsinstruments aus Figur 1 mit geöffneter ersten Endbereichsaufnahme zum Aufnehmen des ersten medizinischen Clips vom ersten Typ;
- Figur 3:: eine Ansicht der Anordnung aus Figur 2 ohne Clip von vorne;
- Figur 4:: eine schematische Ansicht des ersten Applikationsinstruments von vorn mit einem zur ersten Endbereichsaufnahme korrespondierenden ersten medizinischen Clip vom ersten Typ (links) und einem zur ersten Endbereichsaufnahme nicht korrespondierenden zweiten medizinischen Clip vom zweiten Typ (rechts);
- Figur 5:: eine vergrößerte Teilansicht des distalen Endbereichs des ersten Applikationsinstruments mit aufgenommenem ersten medizinischen Clip vom ersten Typ;
- Figur 6:: eine Ansicht des ersten Applikationsinstruments aus Figur 4 von vorn mit darin aufgenommenem ersten medizinischen Clip vom ersten Typ;
- Figur 7:: eine Teilansicht der Anordnung aus Figur 6 in Richtung des Pfeils A;
- Figur 8:: eine Ansicht ähnlich Figur 7, jedoch mit etwas aufeinander zu bewegten Werkzeugelementen;
- Figur 9:: eine schematische perspektivische Ansicht eines distalen Endbereichs des ersten Applikationsinstruments beim Versuch, einen zweiten medizinischen Clip vom zweiten Typ in der ersten Endbereichsaufnahme aufzunehmen;
- Figur 10:: eine Ansicht der Anordnung aus Figur 9 von entsprechend Figur 6;
- Figur 11:: eine schematische Ansicht ähnlich Figur 4, jedoch eines zweiten Applikationsinstruments mit einem ersten medizinischen Clip vom ersten Typ (links) und einem zweiten medizinischen Clip vom zweiten Typ (rechts);
- Figur 12:: eine vergrößerte Teilansicht eines distalen Endbereichs eines weiteren Ausführungsbeispiels eines ersten Applikationsinstruments mit einem weiteren Ausführungsbeispiel eines ersten medizinischen Clips vom ersten Typ;
- Figur 13:: eine vergrößerte perspektivische Teilansicht des distalen Endbereichs des Applikationsinstruments aus Figur 12 mit geöffneter erster Endbereichsaufnahme zum Aufnehmen des ersten medizinischen Clips vom ersten Typ aus Figur 12;
- Figur 14:: eine Ansicht des ersten Applikationsinstruments aus den Figuren 12 und 13 von vorn mit darin aufgenommenem ersten medizinischen Clip vom ersten Typ mit seitlichem Wulst;
- Figur 15:: eine Seitenansicht des ersten medizinischen Clips vom ersten Typ aus den Figuren 12 bis 14;
- Figur 16:: eine Ansicht der Anordnung aus Figur 15 in Richtung des Pfeils A;
- Figur 17:: eine Ansicht der Anordnung aus Figur 15 in Richtung des Pfeils B;
- Figur 18:: eine vergrößerte Teilansicht des ersten Werkzeugelements des ersten Applikationsinstruments aus den Figuren 12 bis 14;
- Figur 19:: eine Ansicht in Richtung des Pfeils C aus Figur 18;
- Figur 20:: eine schematische vergrößerte Ansicht erster Werkzeugelemente eines weiteren Ausführungsbeispiels eines ersten Applikationsinstruments; und
- Figur 21:: eine schematische Ansicht des ersten Applikationsinstruments aus Figur 20 von vorn mit einem zur ersten Endbereichsaufnahme korrespondierenden ersten medizinischen Clip vom ersten Typ (links) und einem zur ersten Endbereichsaufnahme nicht korrespondierenden zweiten medizinischen Clips vom zweiten Typ (rechts).

Ein Ausführungsbeispiel eines Applikationssystems 10 ist schematisch in den Figuren dargestellt. Es dient zum Anlegen medizinischer Clips.

Das Applikationssystem 10 umfasst ein erstes Applikationsinstrument 12 zum Anlegen eines ersten medizinischen Clips 14 von einem ersten Typ. Wie nachfolgend noch eingehend erläutert wird, ist das erste Applikationsinstrument 12 ausgebildet, um einen ersten Clip 14 von einem zweiten medizinischen Clip 16 von einem zweiten Typ zu unterscheiden.

Das erste Applikationsinstrument 12 weist zwei relativ zueinander bewegbare erste Werkzeugelemente 18 auf.

Der erste Clip 14 vom ersten Typ weist einen ersten proximalen Endbereich 20 und zwei erste Verbindungsabschnitte 22 auf. Die zwei ersten Verbindungsabschnitte 22 schließen sich distalseitig an den ersten Endbereich 20 an.

Der erste Endbereich 20 definiert einen ersten Volumenbereich 24. Die zwei ersten Verbindungsabschnitte 22 verbinden den ersten Endbereich 20 mit jeweils einem ersten Klemmarm 26.

Die zwei ersten Werkzeugelemente 18 definieren zwischen sich eine in distaler Richtung geöffnete erste Endbereichsaufnahme 28. In diese kann der erste Endbereich 20 formschlüssig oder im Wesentlichen formschlüssig aufgenommen werden.

An jedem der beiden Werkzeugelemente 18 ist eine erste Verbindungsabschnittsaufnahme 30 ausgebildet. Sie dient zum Aufnehmen mindestens eines Teils eines ersten Aufnahmeabschnitts 32 der ersten Verbindungsabschnitte 22.

Ferner ist an den ersten Werkzeugelementen 18 jeweils ein erstes Unterscheidungselement 34 angeordnet beziehungsweise ausgebildet. Die ersten Unterscheidungselemente 34 sind derart angeordnet oder ausgebildet, dass sie in einen zweiten Volumenbereich 36, welcher von einem zweiten proximalen Endbereich 38 des zweiten Clips 16 vom zweiten Typ definiert wird, eingreifen beziehungsweise hineinragen.

Vom zweiten proximalen Endbereich 38 erstrecken sich in distaler Richtung zwei zweite Verbindungsabschnitte 40 weg in distaler Richtung. Die zweiten Verbindungsabschnitte 40 umfassen jeweils einen zweiten Aufnahmeabschnitt 42.

Der erste Volumenbereich 24 und der zweite Volumenbereich 36 überlappen sich nur teilweise unter der Zwangsbedingung, dass die zweiten Aufnahmeabschnitte 42 jeweils in einer der ersten Verbindungsabschnittsaufnahmen 30 der zwei ersten Werkzeugelemente 18 aufgenommen sind. Dadurch wird verhindert, dass der zweite Clip 16 vom zweiten Typ mit seinem zweiten proximalen Endbereich 38 in der ersten Endbereichsaufnahme 28 aufgenommen werden kann.

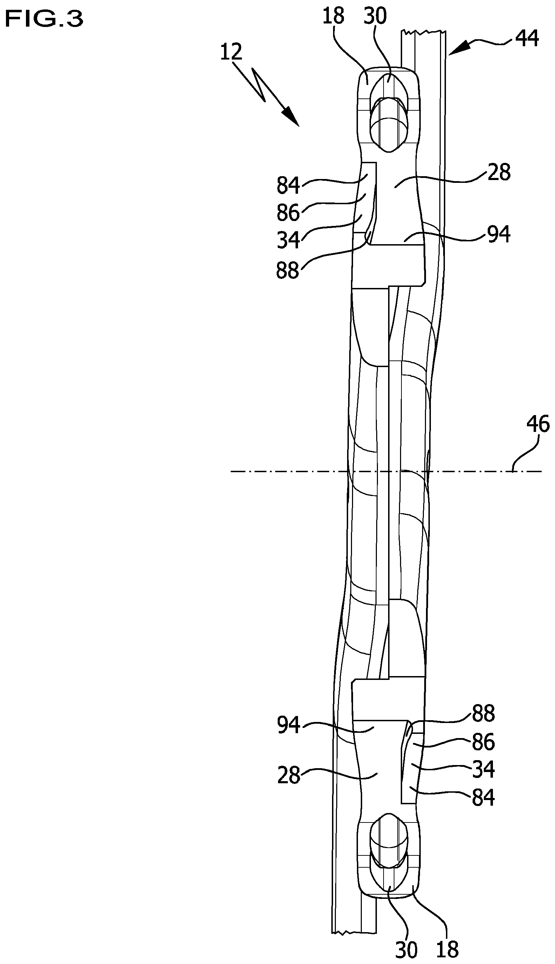

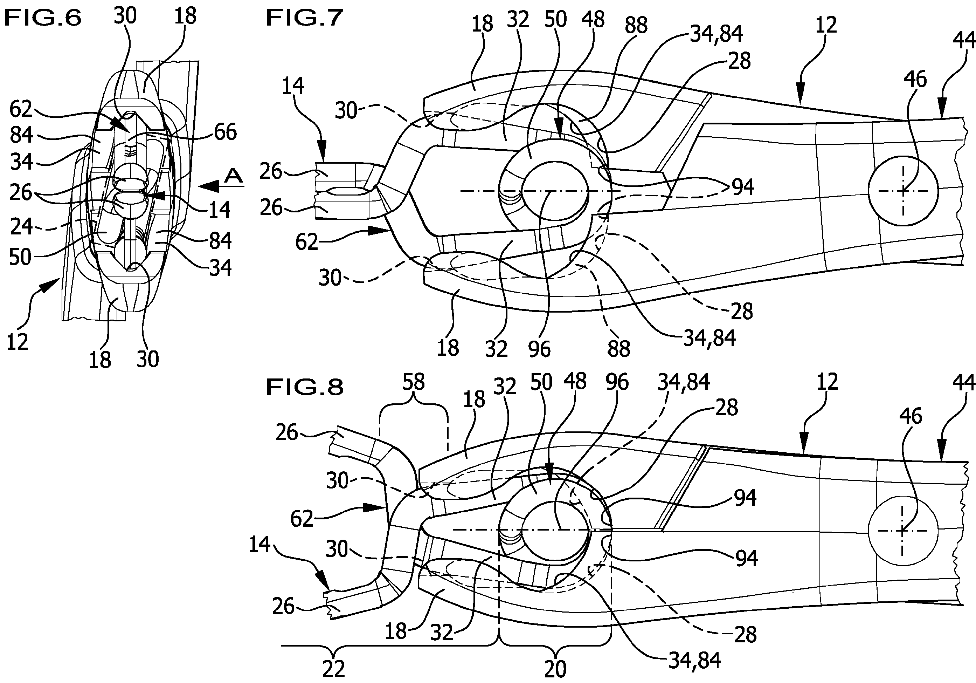

Das erste Applikationsinstrument 12 umfasst eine erste Betätigungseinrichtung 44 zum Bewegen, nämlich zum Verschwenken, der zwei ersten Werkzeugelemente 18 relativ zueinander von einer ersten Aufnahmestellung, wie sie schematisch in Figur 3 dargestellt ist und in welcher ein zum ersten Applikationsinstrument 12 korrespondierender erster medizinischer Clip 14 vom ersten Typ mit seinem ersten Endbereich 20 in der ersten Endbereichsaufnahme 28 aufnehmbar ist, in eine erste Applikationsstellung, die schematisch in Figur 8 dargestellt ist und in welcher die zwei ersten Werkzeugelemente 18 etwas aufeinander zu und die ersten Klemmarme 26 voneinander weg bewegt sind, so dass der erste Clip 14 vom ersten Typ appliziert werden kann.

Die ersten Werkzeugelemente 18 des ersten Applikationsinstruments 12 umgreifen den ersten proximalen Endbereich 20 in der ersten Aufnahmestellung und in der ersten Applikationsstellung teilweise.

Die zwei ersten Werkzeugelemente 18 sind bei dem in den Figuren dargestellten ersten Applikationsinstrument 12 um eine gemeinsame erste Schwenkachse 46 relativ zueinander verschwenkbar.

Die ersten Aufnahmeabschnitte 32 schließen sich bei den in den Figuren schematisch dargestellten ersten Clips 14 vom ersten Typ direkt beziehungsweise im Wesentlichen direkt an den ersten proximalen Endbereich 20 an. In entsprechender Weise schließen sich die zweiten Aufnahmeabschnitte 42 direkt beziehungsweise im Wesentlichen direkt an den zweiten proximalen Endbereich 38 an.

Der erste proximale Endbereich 20 umfasst ein erstes vorspannendes Element 48 in Form einer ersten Schraubenfeder 50 mit etwa eineinhalb Windungen.

Der zweite Endbereich 38 umfasst in entsprechender Weise ein zweites vorspannendes Element 52, welches in Form einer zweiten Schraubenfeder 54 mit ebenfalls etwa eineinhalb Windungen ausgebildet ist.

Die vorspannenden Elemente 48 und 52 sind bei dem beschriebenen Ausführungsbeispiel durch einen Kaltumformprozess ausgebildet.

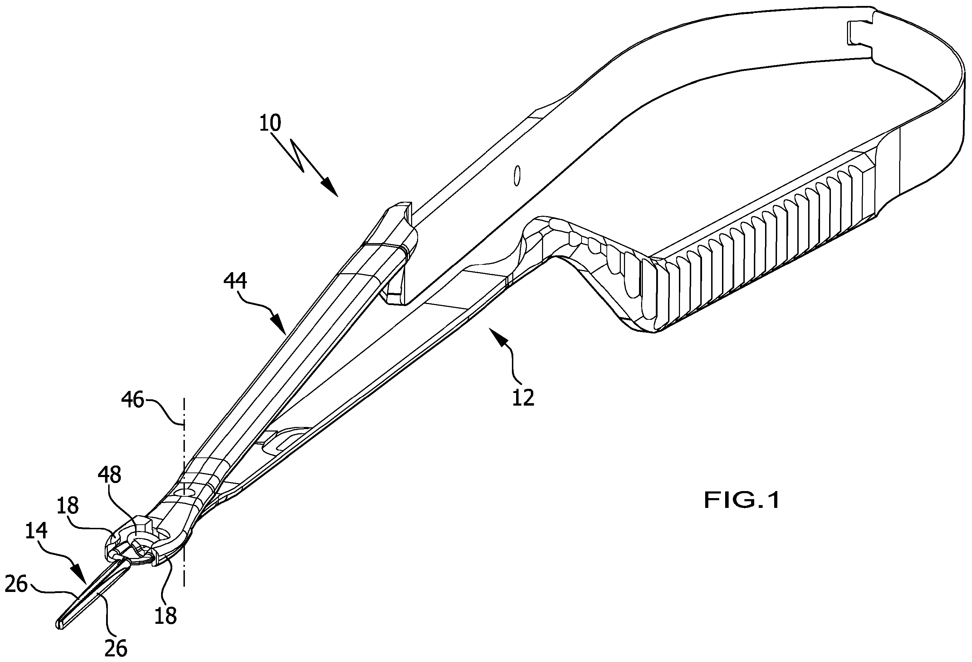

Die ersten Klemmarme 26 liegen in einer ersten Grundstellung des ersten Clips 14 vom ersten Typ aneinander an, wie dies schematisch insbesondere in Figur 1 dargestellt ist. Die Klemmarme 26 sind entgegen der Wirkung des ersten vorspannenden Elements 48 von der ersten Grundstellung in eine beliebige erste Öffnungsstellung voneinander weg bewegbar. Eine solche erste Öffnungsstellung ist beispielsweise schematisch in Figur 8 dargestellt.

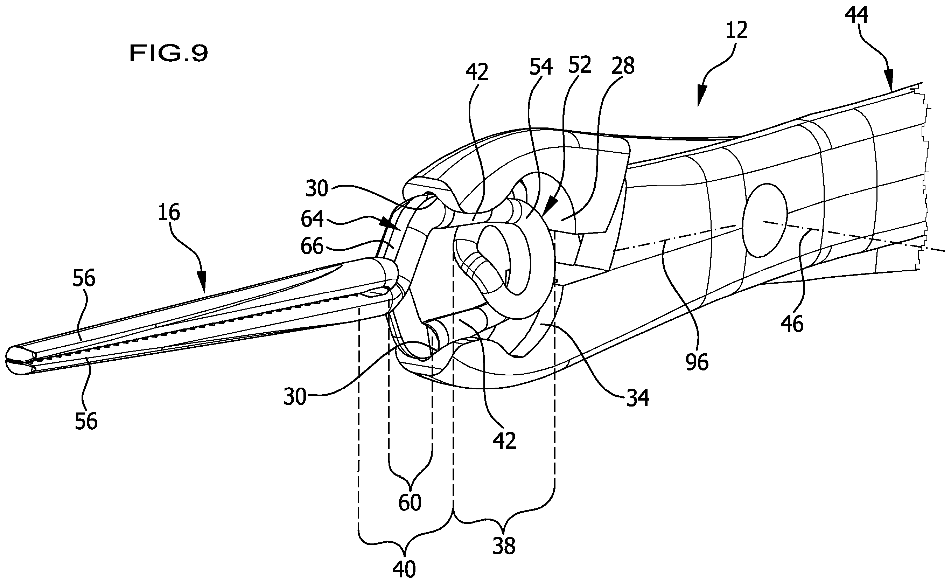

Der zweite Clip 16 vom zweiten Typ weist zwei zweite Klemmarme 56 auf, die in einer Grundstellung, wie schematisch in Figur 9 dargestellt, aneinander anliegen und entgegen der Wirkung des zweiten vorspannenden Elements 52 von der zweiten Grundstellung in eine beliebige zweite Öffnungsstellung voneinander bewegbar sind, also analog zu einer Stellung des ersten Clips 14, wie sie in Figur 8 schematisch dargestellt ist.

Bei dem in den Figuren dargestellten Ausführungsbeispielen des ersten Clips 14 vom ersten Typ und des zweiten Clips 16 vom zweiten Typ kreuzen sich die ersten Verbindungsabschnitte 22 in einem ersten Schlussbereich 58. In analoger Weise kreuzen sich die zweiten Verbindungsabschnitte 40 in einem zweiten Schlussbereich des zweiten medizinischen Clips 16 vom zweiten Typ.

Der erste Schlussbereich 58 ist distalseitig der ersten Aufnahmeabschnitte 32 angeordnet beziehungsweise ausgebildet. In analoger Weise ist der zweite Schlussbereich 60 distalseitig der zweiten Aufnahmeabschnitte 42 angeordnet oder ausgebildet.

Die Schlussbereiche 58 und 60 sind in Form von Durchsteckschlüssen 62 beziehungsweise 64 ausgebildet. Einer der Verbindungsabschnitte 22 beziehungsweise 40 weist dabei eine Schlussbereichsdurchbrechung 66 auf, die vom anderen Verbindungsabschnitt 22 beziehungsweise 40 durchsetzt ist.

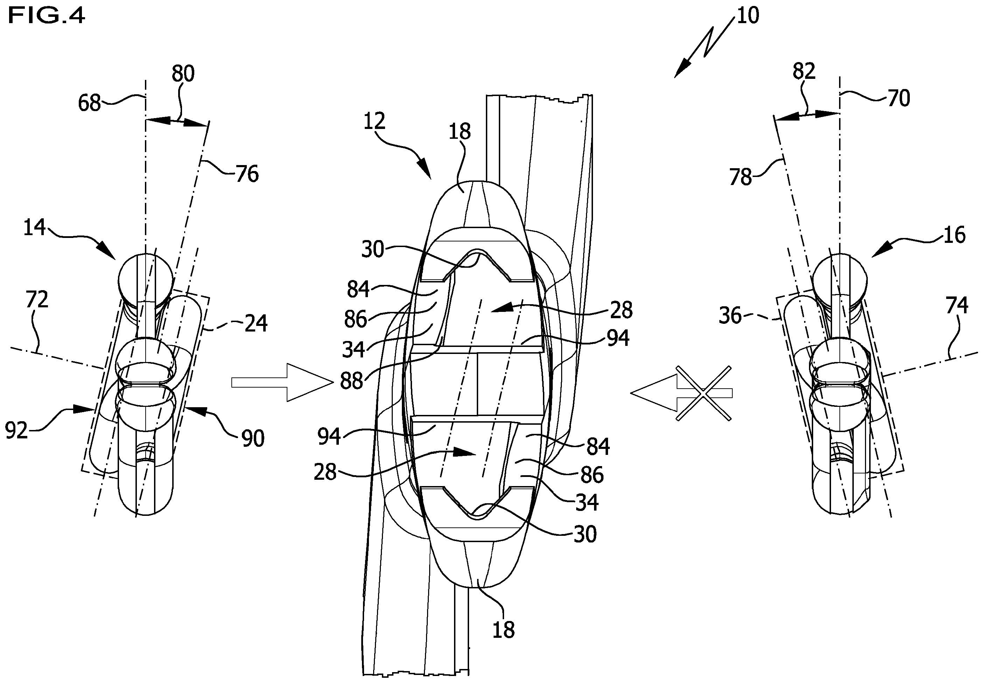

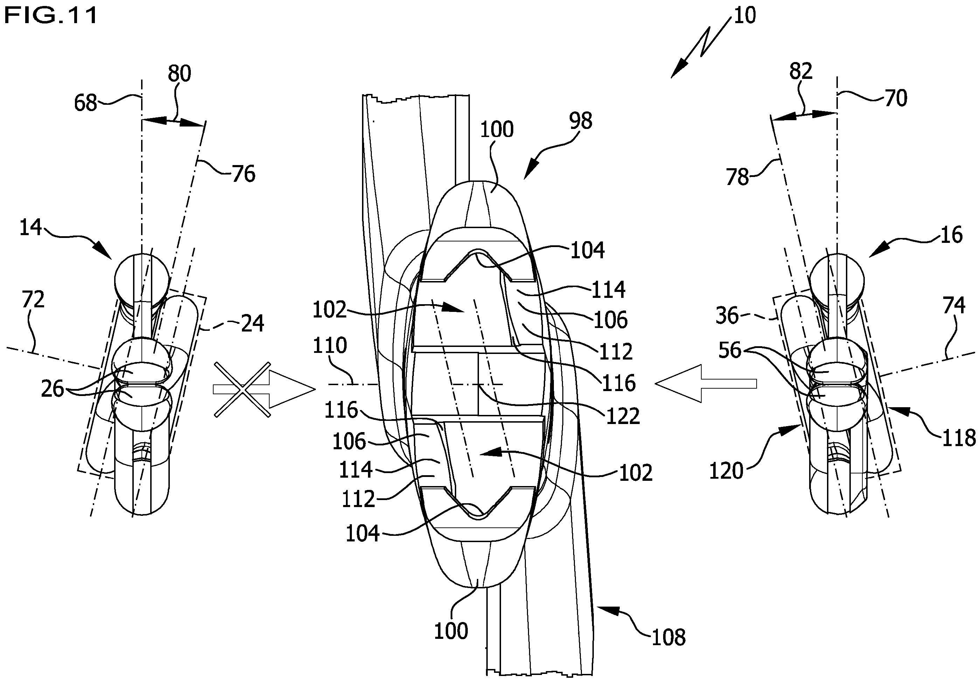

Mit Bezug zu den Figuren 4 und 11 definiert der erste medizinische Clip 14 vom ersten Typ eine erste Clipebene 68. Diese wird durch die beiden ersten Aufnahmeabschnitte 32 aufgespannt.

In analoger Weise definiert der zweite medizinische Clip 16 eine zweite Clipebene 70.

Die erste Schraubenfeder 50 definiert eine erste Schraubenfederlängsachse 72, um die die erste Schraubenfeder 50 im Uhrzeigersinn gewickelt ist.

Die zweite Schraubenfeder 54 definiert eine zweite Schraubenfederlängsachse 74, um die die zweite Schraubenfeder 54 im Gegenuhrzeigersinn gewickelt ist.

Die erste Schraubenfederlängsachse 72 verläuft senkrecht zu einer ersten Schraubenfederebene 76. Ebenso verläuft die zweite Schraubenfederlängsachse 74 senkrecht zu einer zweiten Schraubenfederebene 78.

Die erste Schraubenfederebene 76 ist relativ zur ersten Clipebene 68 um einen ersten Neigungswinkel 80 geneigt. Die zweite Schraubenfederebene 78 ist relativ zur zweiten Clipebene 70 um einen zweiten Neigungswinkel 82 geneigt.

Der erste Neigungswinkel 80 weist bei der im Uhrzeigersinn gewickelten ersten Schraubenfeder 50 einen positiven Wert auf, welcher in einem Bereich zwischen 0° und etwa 40° liegt. Bei dem in den Figuren dargestellten Ausführungsbeispiel des ersten Clips 14 liegt der Wert des Neigungswinkels 80 in einem Bereich zwischen 0° und etwa 20°.

Der zweite Neigungswinkel 82 weist bei der im Gegenuhrzeigersinn gewickelten zweiten Schraubenfeder 54 einen negativen Wert auf, welcher in einem Bereich zwischen 0° und etwa -40° liegt. Bei dem in den Figuren dargestellten Ausführungsbeispiel liegt der zweite Neigungswinkel 82 in einem Bereich zwischen 0° und -20°.

Bei dem Applikationssystem 10 ist das erste Applikationsinstrument 12 korrespondierend zum ersten medizinischen Clip 14 vom ersten Typ mit einer im Uhrzeigersinn gewickelten ersten Schraubenfeder 50 ausgebildet.

Die ersten Unterscheidungselemente 34 sind in Form erster Kodierelemente 84 ausgebildet. Sie sind derart angeordnet und ausgebildet, dass in der ersten Endbereichsaufnahme 28 des ersten Applikationsinstruments 12 ausschließlich ein zu den ersten Kodierelementen 84 korrespondierender erster medizinischer Clip 14 vom ersten Typ aufgenommen werden kann. Dies ist schematisch in Figur 6 dargestellt. Ferner zeigt Figur 4 links den ersten Clip 14 vom ersten Typ, welcher in die erste Endbereichsaufnahme 28 einführbar ist.

Die ersten Unterscheidungselemente 34 wirken jedoch auch mit anderen Clips zusammen, also insbesondere auch mit den zweiten medizinischen Clips 16 vom zweiten Typ.

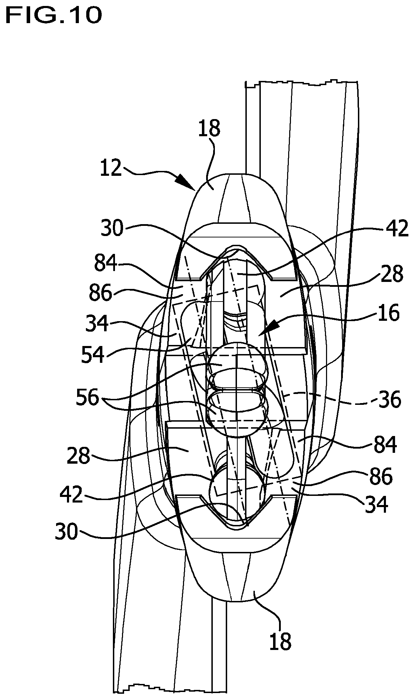

Im Zusammenwirken mit einem zweiten medizinischen Clip 16 verhindern die ersten Kodierelemente 84 das Einführen des zweiten Endbereichs 38 in die erste Endbereichsaufnahme 28. In Figur 4 rechts ist der zweite medizinische Clip 16 vom zweiten Typ dargestellt, dessen zweiter Endbereich 38 nicht zur ersten Endbereichsaufnahme 28 korrespondiert. Diese ermöglicht lediglich die Aufnahme des ersten Volumenbereichs 24, der vom ersten Endbereich 20 definiert wird, wenn die ersten Aufnahmeabschnitte 32 in den ersten Verbindungsabschnittsaufnahmen 30 der ersten Werkzeugelemente 18 aufgenommen sind. Dagegen kollidieren die Kodierelemente 84 mit dem zweiten Volumenbereich 36, der vom zweiten Endbereich 38 des zweiten medizinischen Clip 16 vom zweiten Typ definiert wird, wenn die zweiten Aufnahmeabschnitte 42 in die ersten Verbindungsabschnittsaufnahmen 30 eingreifen. Dies ist unmittelbar erkennbar aus den Figuren 4 und 10.

Es sei angemerkt, dass Figur 10 eine Situation zeigt, wie sie real nicht möglich ist, da der zweite Endbereich 38 und die Kodierelemente 84 überlappen. Hier ist gut erkennbar, also insbesondere in Figur 10, dass das Kodierelement 84 in den zweiten Volumenbereich 36 unter der beschriebenen Zwangsbedingung, dass die zweiten Aufnahmeabschnitte 42, die in den ersten Verbindungsabschnittsaufnahmen 30 aufgenommen sind, eingreift. Wie bereits erläutert ist diese Kollision real nicht möglich. Vielmehr führt diese Ausgestaltung des ersten Applikationsinstruments 12 im Zusammenwirken mit dem zweiten medizinischen Clip 16 vom zweiten Typ dazu, dass dieser mit seinem zweiten Endbereich 38 aus der ersten Endbereichsaufnahme 28 herausgedrückt wird.

Das erste Kodierelement 84 ist in Form eines ersten Kodiervorsprungs 86 ausgebildet, welcher die erste Endbereichsaufnahme 28 abschnittsweise seitlich begrenzt.

Das erste Applikationsinstrument 12 umfasst zwei zusammenwirkende erste Kodierelemente 84, die seitliche Anlageflächen 88 für voneinander weg weisende Seiten 90 und 92 des in der ersten Endbereichsaufnahme 28 aufgenommenen ersten Endbereichs 20 bilden.

An jedem der beiden ersten Werkzeugelemente 18 ist in der beschriebenen Weise ein Kodierelement 84 angeordnet beziehungsweise ausgebildet.

Ferner steht das erste Kodierelement 84 am einen der beiden ersten Werkzeugelemente 18 in distaler Richtung über ein proximales Ende 94 des die erste Endbereichsaufnahme 28 begrenzenden anderen ersten Werkzeugelements 18 vor.

Die zwei ersten Werkzeugelemente 18 sind in ihrer Form identisch ausgebildet und im Bereich der ersten Endbereichsaufnahme 28 durch Drehung um 180° bezogen auf eine erste Längsachse 96 der ersten Endbereichsaufnahme 28 ineinander überführbar.

Die beiden Clips 14 und 16 sind jeweils in Form von Aneurysmenclips ausgebildet.

Das in den Figuren schematisch dargestellte Applikationssystem 10 umfasst ferner ein zweites Applikationsinstrument 98, um den zweiten medizinischen Clip 16 vom zweiten Typ anzulegen.

Das zweite Applikationsinstrument 98 weist zwei relativ zueinander bewegbare zweite Werkzeugelemente 100 auf. Die zwei zweiten Werkzeugelemente 100 definieren zwischen sich eine in distaler Richtung geöffnete zweite Endbereichsaufnahme 102, in die der zweite proximale Endbereich 38 formschlüssig oder im Wesentlichen formschlüssig aufgenommen werden kann.

An den zweiten Werkzeugelementen 100 ist jeweils eine zweite Verbindungsabschnittsaufnahme 104 ausgebildet. Diese sind aufeinander zu weisend ausgerichtet und dafür vorgesehen, jeweils einen der zweiten Aufnahmeabschnitte 42 des zweiten medizinischen Clips 16 vom zweiten Typ aufzunehmen.

An den zwei zweiten Werkzeugelementen 100 sind zweite Unterscheidungselemente 106 derart angeordnet beziehungsweise ausgebildet, dass sie in den ersten Volumenbereich 24 unter der Zwangsbedingung eingreifen, dass die ersten Aufnahmeabschnitte 32 in jeweils einer der zweiten Verbindungsabschnittsaufnahmen 104 aufgenommen sind. Mit anderen Worten ist das zweite Applikationsinstrument 98 derart ausgebildet, dass es unter der beschriebenen Zwangsbedingung lediglich geeignet ist, den zweiten medizinischen Clip 16 vom zweiten Typ mit dessen zweitem Endbereich 38 in der zweiten Endbereichsaufnahme 102 aufzunehmen, nicht jedoch den ersten Endbereich 20 des ersten medizinischen Clips 14 vom ersten Typ.

Die Applikationsinstrumente 12 und 98 sind daher geeignet, die Clips 14 und 16 mechanisch und insbesondere automatisch voneinander zu unterscheiden.

Das zweite Applikationsinstrument 98 umfasst eine zweite Betätigungseinrichtung 108 zum Bewegen, bei dem in Figur 11 dargestellten Ausführungsbeispiel zum Verschwenken, der zweiten Werkzeugelemente 100 relativ zueinander um eine zweite Schwenkachse 110. Mit der Betätigungseinrichtung 108 können die zwei zweiten Werkzeugelemente 100 relativ zueinander von einer zweiten Aufnahmestellung, wie sie schematisch in Figur 11 dargestellt ist, und in welcher ein zum zweiten Applikationsinstrument 98 korrespondierender zweiter medizinischer Clip 16 vom zweiten Typ in seinem zweiten Endbereich 38 in der zweiten Endbereichsaufnahme 102 aufnehmbar ist, in eine zweite Applikationsstellung, in welcher die zwei zweiten Werkzeugelemente 100 aufeinander zu und die zweiten Klemmarme 56 voneinander weg bewegt sind, um den zweiten Clip 16 vom zweiten Typ applizieren zu können.

Die zweiten Werkzeugelemente 100 umgreifen den zweiten proximalen Endbereich 38 in der zweiten Aufnahmestellung und in der zweiten Applikationsstellung teilweise.

Wie bereits oben erläutert und aus Figur 11 unmittelbar erkennbar, ist das zweite Applikationsinstrument 98 korrespondierend zum zweiten medizinischen Clip 16 vom zweiten Typ ausgebildet, welcher eine im Gegenuhrzeigersinn gewickelte zweite Schraubenfeder 54 aufweist.

Das zweite Unterscheidungselement 106 ist analog zum ersten Unterscheidungselement 34 in Form eines zweiten Kodierelements 112 ausgebildet, welches mit beliebigen medizinischen Clips zusammenwirkt. Das zweite Kodierelement 112 ist jedoch im Bereich der zweiten Endbereichsaufnahme 102 des zweiten Applikationsinstruments 98 derart angeordnet oder ausgebildet, dass ausschließlich der zum zweiten Kodierelement 112 korrespondierende zweite medizinische Clip 16 vom zweiten Typ formschlüssig oder im Wesentlichen formschlüssig in der zweiten Endbereichsaufnahme 102 aufnehmbar ist.

Auch das zweite Kodierelement 112 ist in Form eines zweiten Kodiervorsprungs 114 ausgebildet, welcher die zweite Endbereichsaufnahme 102 abschnittsweise seitlich begrenzt.

Das in den Figuren schematisch dargestellte zweite Applikationsinstrument 98 weist zwei zweite Kodierelemente 112 auf. Diese definieren aufeinander zu weisende seitliche Anlageflächen 116 für voneinander weg weisende Seiten 118 und 120 des in der zweiten Endbereichsaufnahme 102 aufgenommenen zweiten Endbereichs 38.

Wie oben in Verbindung mit Figur 7 und dem ersten Applikationsinstrument 12 erläutert, sind auch die zweiten Kodierelemente 112 am jeweiligen zweiten Werkzeugelement 100 in distaler Richtung über ein proximales Ende des die zweite Endbereichsaufnahme 102 begrenzenden anderen zweiten Werkzeugelement 100 vorstehend ausgebildet.

Auch die zwei zweiten Werkzeugelemente 100 sind im Bereich der zweiten Endbereichsaufnahme 102 durch Drehung um 180° bezogen auf eine zweite Längsachse 122, welche parallel zur zweiten Clipebene 70 verläuft, ineinander überführbar.

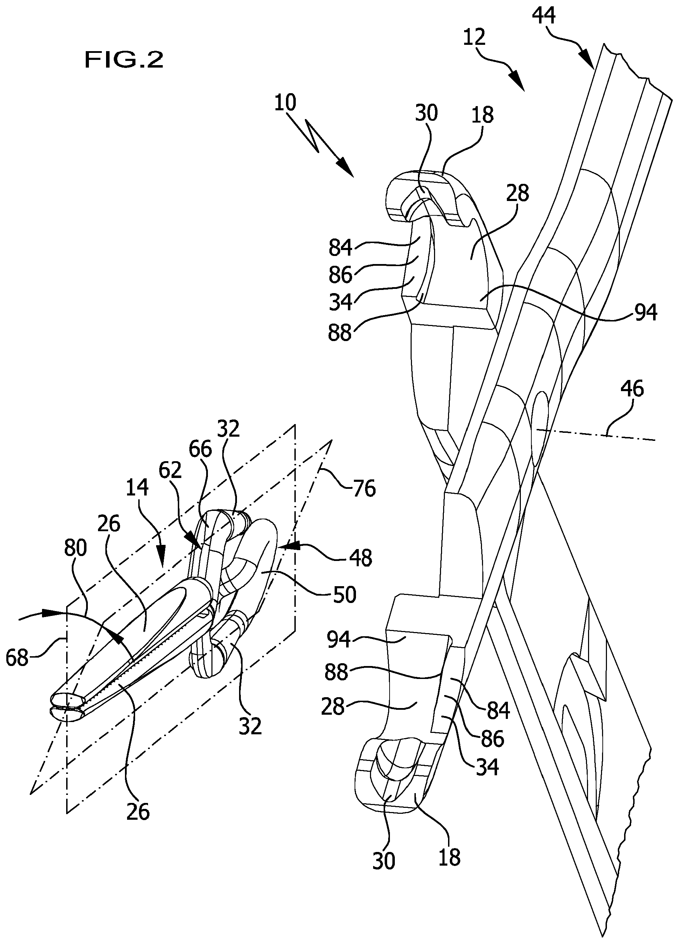

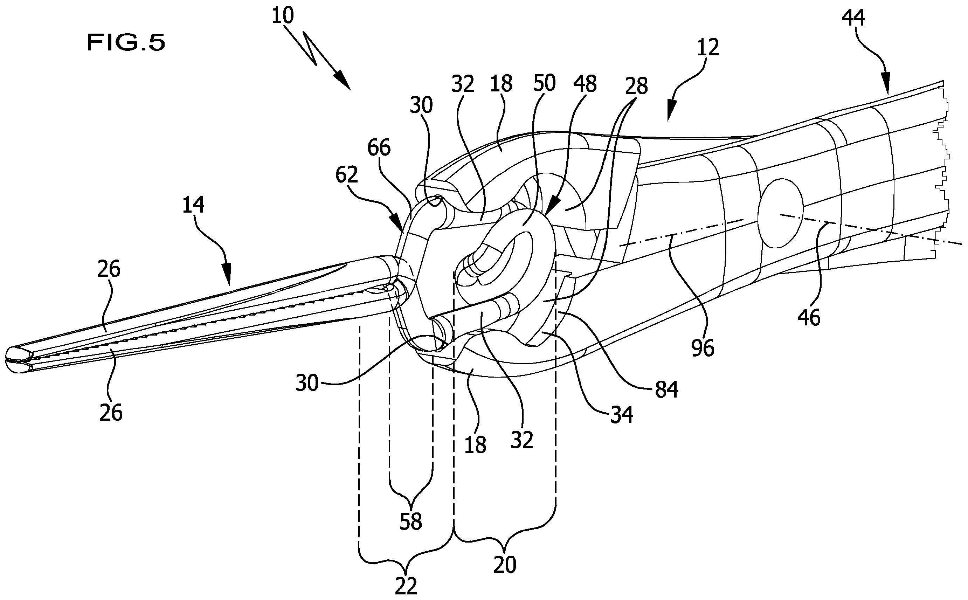

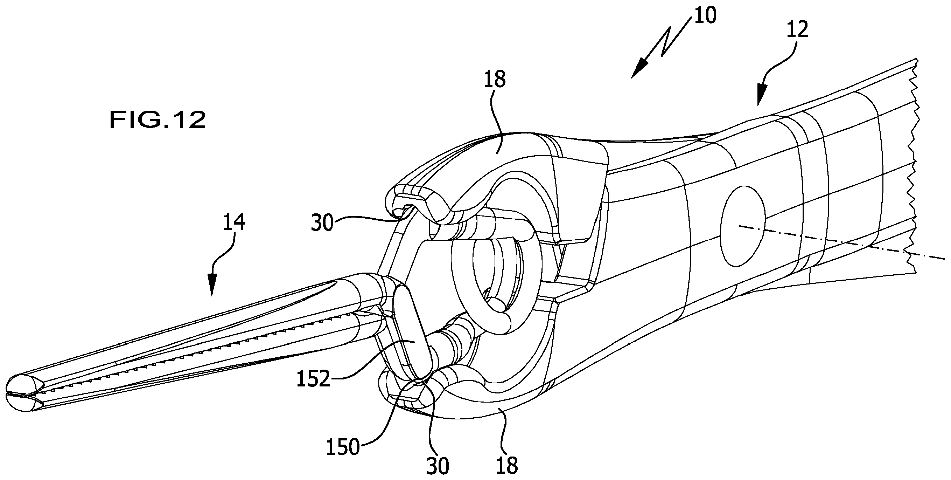

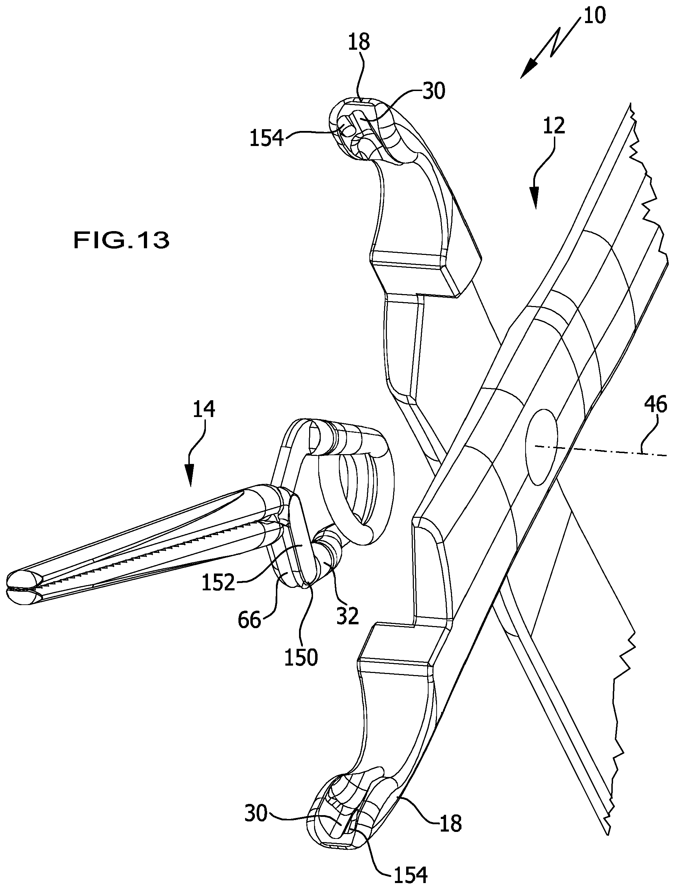

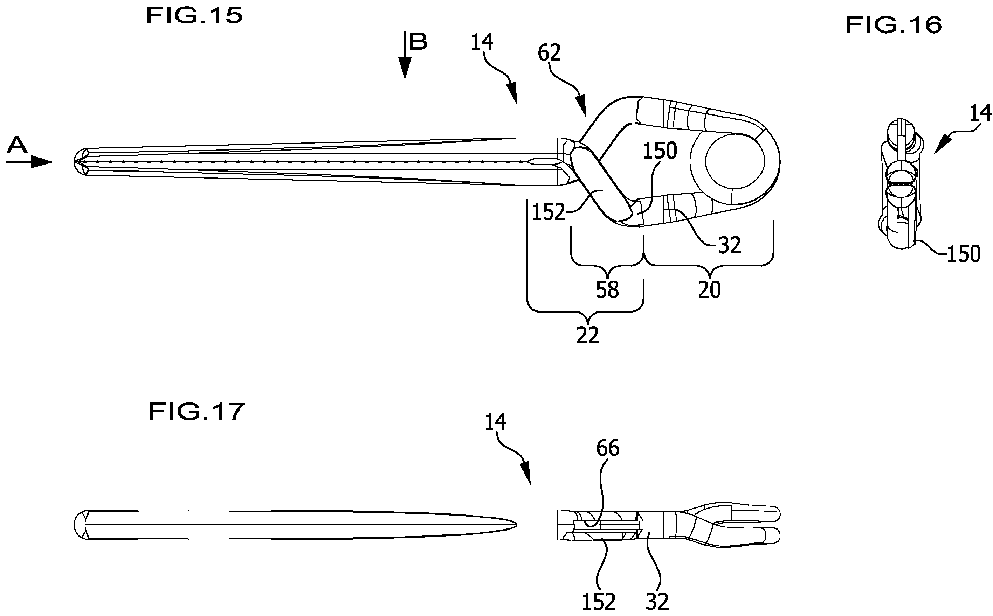

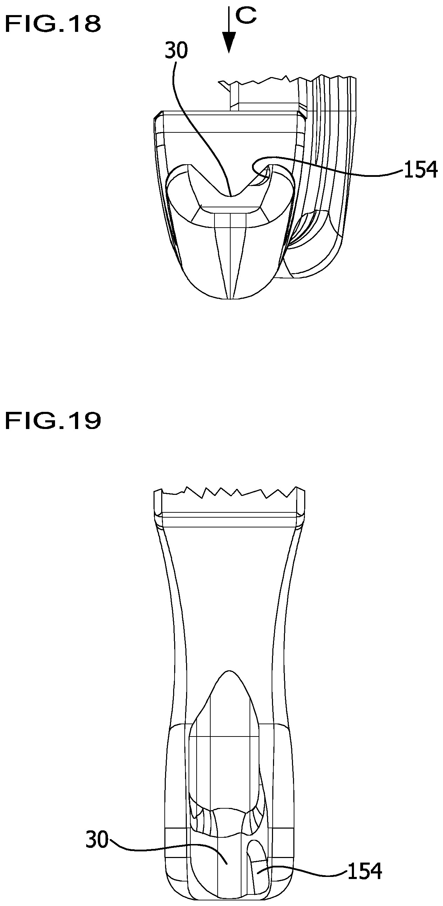

In den Figuren 12 bis 19 ist schematisch ein weiteres Ausführungsbeispiel eines Applikationssystems 10 dargestellt. Es entspricht bis auf die nachfolgend näher beschriebenen Modifikationen dem oben in Verbindung mit den Figuren 1 bis 11 beschriebenen Ausführungsbeispiel des Applikationssystems 10. Die in den Figuren 12 bis 19 verwendeten Bezugszeichen stimmen mit den in den Figuren 1 bis 11 verwendeten Bezugszeichen für entsprechende Teile und Komponenten überein.

In den Figuren 12 bis 14 ist schematisch ein erstes Applikationsinstrument 12 im Zusammenwirken mit einem zu diesem korrespondierenden ersten Clip 14 vom ersten Typ dargestellt. Der Unterschied des ersten Clips 14 vom ersten Typ gemäß dem Ausführungsbeispiel der Figuren 12 bis 19 vom ersten Clip 14 des ersten Typs gemäß den Figuren 1 bis 11 besteht darin, dass im Übergangsbereich vom ersten Endbereich 20 zum ersten Verbindungsabschnitt 22, welcher die Schlussbereichsdurchbrechung 66 definiert, ein seitlich abstehender Wulst 150 ausgebildet ist. Dieser Wulst 150 entsteht insbesondere beim Herstellen dieses ersten Clips 14 vom ersten Typ, nämlich dann, wenn der Clip 14 im Bereich des Durchsteckschlusses 62 nicht durch ein generatives Herstellungsverfahren ausgebildet ist, sondern durch Anlöten oder Anschweißen eines seitlichen Schlussplättchens 152, welches zum seitlichen Verschließen der bei Umformung eines Rohlings, aus dem der Clip 14 ausgebildet wird, gebildeten Schlussbereichsdurchbrechung 66 in einen Rücksprung eingesetzt ist. Der Wulst 150 kann insbesondere in Form einer Schweißraupe ausgebildet sein.

Der Wulst 150 ist bei dem in den Figuren 12 bis 19 dargestellten Ausführungsbeispiel des ersten Clips 14 vom ersten Typ am Ende des ersten Aufnahmeabschnitts 32 ausgebildet und steht seitlich in einer Richtung parallel zur ersten Schwenkachse 46 vom ersten Aufnahmeabschnitt 32 ab.

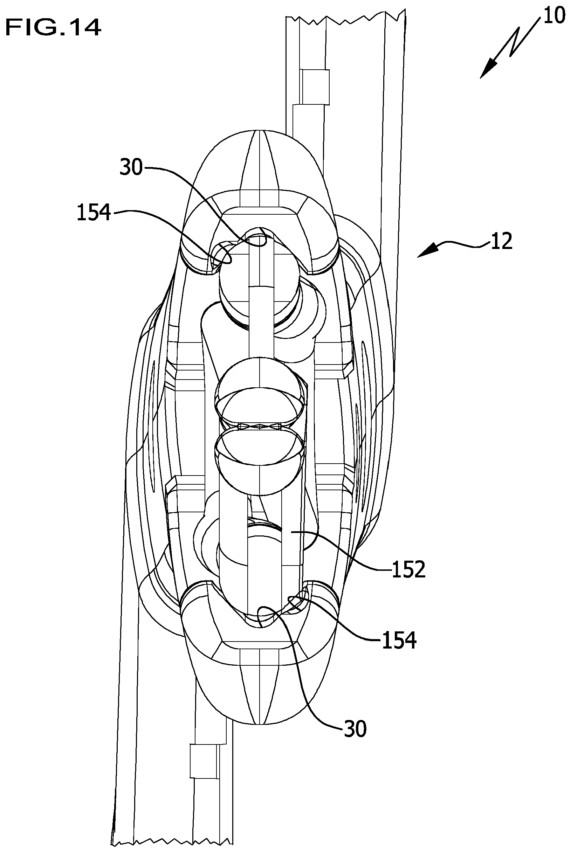

Der Wulst 150 verhindert, dass der erste Clip 14 vom ersten Typ gemäß den Figuren 12 bis 18 vom ersten Applikationsinstrument 12 gemäß den Figuren 1 bis 11 in definierter Weise und insbesondere mit dem Aufnahmeabschnitt 32 von dessen ersten Verbindungsabschnittsaufnahmen 30 vollständig aufgenommen werden kann. Eine Anlage der Aufnahmeabschnitte 32 in den Verbindungsabschnittsaufnahmen 30, wie dies in Figur 6 gut zu erkennen ist, ist bei dem Clip 14 mit Wulst 150 nicht möglich. Damit dies möglich wird, ist am ersten Applikationsinstrument 12 gemäß dem Ausführungsbeispiel der Figuren 12 bis 19 eine Wulstaussparung 154 an den ersten Werkzeugelementen 18 des ersten Applikationsinstruments 12 ausgebildet. Die Wulstaussparung 154 bildet eine seitliche Vertiefung der ersten Verbindungsabschnittsaufnahme 30, und zwar auf der Seite, auf der der Wulst 150 des ersten Clips 14 vom ersten Typ seitlich vorsteht. Somit ermöglicht es die Wulstaussparung 154, dass auch der erste Clip 14 vom ersten Typ gemäß dem Ausführungsbeispiel der Figuren 12 bis 19 vollständig und in definierter Weise vom ersten Applikationsinstrument 12 gemäß dem Ausführungsbeispiel der Figuren 12 bis 18 aufgenommen werden kann, wie dies schematisch insbesondere in den Figuren 12 und 14 dargestellt ist.

In analoger Weise wie der erste Clip 14 vom ersten Typ gemäß dem Ausführungsbeispiel der Figuren 12 bis 19 weist auch ein zweiter, in den Figuren nicht dargestellter Clip vom zweiten Typ einen Wulst auf. Lediglich dieser Wulst unterscheidet den zweiten Clip vom zweiten Typ gemäß dem Ausführungsbeispiel der Figuren 12 bis 19 vom zweiten Clip 16 des zweiten Typs gemäß dem Ausführungsbeispiel der Figuren 1 bis 11.

Damit auch dieser zweite Clip vom zweiten Typ mit Wulst in den Verbindungsabschnittsaufnahmen eines zweiten, in den Figuren ebenfalls nicht dargestellten Applikationsinstruments aufgenommen werden kann, sind auch an den zweiten Endbereichsaufnahmen dieses zweiten Applikationsinstruments den Wulstaussparungen 154 entsprechende Wulstaussparung für den Wulst am zweiten Clip vom zweiten Typ ausgebildet ist.

Die in Verbindung mit dem Ausführungsbeispiel des Applikationssystems 10 gemäß den Figuren 12 bis 19 beschriebene Wulstaussparung 154 kann auch bei beliebigen Applikationsinstrumenten zum Applizieren medizinischer Clips vorgesehen werden, wenn die Clips wie beschrieben seitlich die Schlussbereichsdurchbrechung verschließende Schlussplättchen aufweisen, die durch Schweißen oder Löten mit dem Clip im jeweiligen Verbindungsabschnitt verbunden sind. Ein solches Applikationsinstrument kann, muss aber kein Bestandteil eine Applikationssystems 10 sein, um wie beschrieben unterschiedlich geformte Clips voneinander unterscheiden zu können.

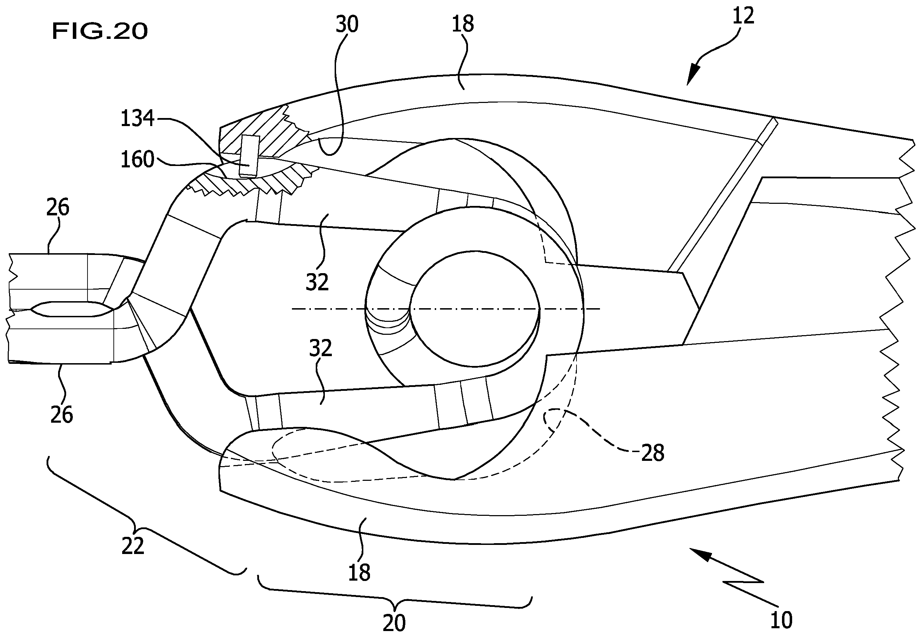

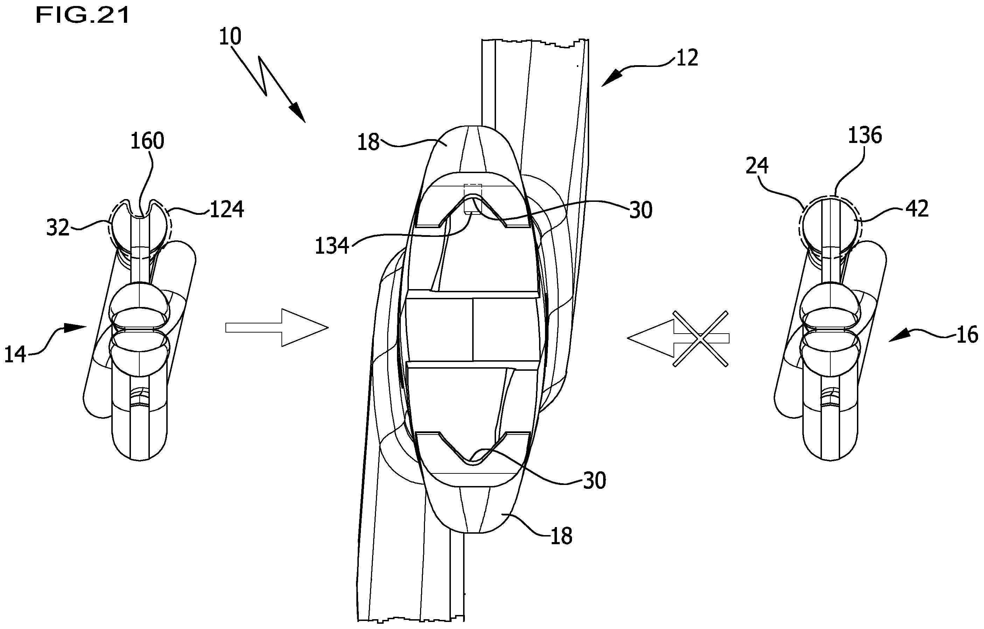

Ein weiteres Ausführungsbeispiel eines Applikationssystems 10 ist schematisch in den Figuren 20 und 21 dargestellt.

Dieses Applikationssystem 10 für medizinische Clips umfasst mindestens ein erstes Applikationsinstrument 12 zum Anlegen eines ersten Typs eines medizinischen Clips 14. In den Figuren 20 und 21 ist nur ein erstes Applikationsinstrument 12 teilweise dargestellt.

Das Applikationssystem 10 umfasst ferner mindestens einen ersten medizinischen Clip 14 vom ersten Typ. In Figur 20 und in Figur 21 links ist nur ein einziger erster medizinischer Clip 14 beispielhaft dargestellt.

Das erste Applikationsinstrument 12 umfasst zwei relativ zueinander bewegbare erste Werkzeugelemente 18, wobei der mindestens eine erste medizinische Clip 14 vom ersten Typ einen ersten proximalen Endbereich 20 und zwei erste Verbindungsabschnitte 22 aufweist, welche sich distalseitig an den ersten proximalen Endbereich 20 anschließen und den ersten proximalen Endbereich 20 mit zwei relativ zueinander bewegbaren Klemmarmen 26 des mindestens einen ersten Clips 14 vom ersten Typ verbinden. Mindestens einer der zwei ersten Verbindungsabschnitte 22 definiert einen ersten Volumenbereich 124.

Die zwei ersten Werkzeugelemente 18 umfassen zwischen sich eine in distaler Richtung geöffnete erste Endbereichsaufnahme 28 zum formschlüssigen oder im Wesentlichen formschlüssigen Aufnehmen des ersten proximalen Endbereichs 20 und jeweils eine erste Verbindungsabschnittsaufnahme 30 zum mindestens teilweisen Aufnehmen jeweils eines ersten Aufnahmeabschnitts 32 der ersten Verbindungsabschnitte 22.

Das Applikationssystem 12 umfasst ferner mindestens einen zweiten medizinischen Clip 16 von einem zweiten Typ. In Figur 21 rechts ist nur ein einziger zweite medizinischer Clip 16 beispielhaft dargestellt. Der mindestens eine zweite medizinische Clip 16 umfasst zwei Verbindungsabschnitte 40, welche jeweils einen Aufnahmeabschnitt 42 definierten.

An mindestens einem der zwei ersten Werkzeugelemente 18 ist mindestens ein erstes Unterscheidungselement 134 derart angeordnet oder ausgebildet ist, dass es in einen zweiten Volumenbereich 136 eingreift, welcher von mindestens einem der zwei Aufnahmeabschnitte 42 definiert wird.

Der erste Volumenbereich 124 und der zweite Volumenbereich 136 überlappen nur teilweise unter der Zwangsbedingung, dass der andere der zwei zweiten Aufnahmeabschnitte in einer ersten Verbindungsabschnittsaufnahme 30 der zwei ersten Werkzeugelemente 18 und der proximale Endbereich 20 in der ersten Endbereichsaufnahme 28 aufgenommen sind.

Der Clip 14 unterscheidet sich vom Clip 16 dieses Ausführungsbeispiels dadurch, dass an einem der zwei ersten Aufnahmeabschnitte 32 eine Unterscheidungselementaufnahme 160 ausgebildet ist, in welche das Unterscheidungselement 134 am ersten Applikationsinstrument 12 eingreift, wenn der der proximale Endbereich 20 in der Endbereichsaufnahme 28 und der andere der zwei ersten Aufnahmeabschnitte 32 in der anderen Verbindungsabschnittsaufnahme 30 des anderen Werkzeugelements 18 aufgenommen sind.

Da an keinem der zweiten Verbindungsabschnitte 42 des zweiten Clips 16 eine Unterscheidungselementaufnahme ausgebildet ist, verhindert das bei dem in den Figuren dargestellten Ausführungsbeispiel in Form eines abstehenden Stifts ausgebildete Unterscheidungselement 134, dass einer der zweiten Verbindungsabschnitte 42 in der Verbindungsabschnittsaufnahme 30, an der das Unterscheidungselement 134 angeordnet ist, eintauchen kann. So wird verhindert, dass mit dem ersten Applikationsinstrument 12 der Clip 16 gehandhabt werden kann. Mit dem ersten Applikationsinstrument 12 können somit die beiden Clips 14 und 16 voneinander unterschieden werden.

Die oben beschriebenen Ausführungsbeispiele von Applikationssystemen 10 für medizinische Clips ermöglichen auf einfache Weise eine sichere Unterscheidung mechanisch unterschiedlicher medizinischer Clips 14 und 16 voneinander. So können insbesondere unterschiedlich geformte Endbereiche 20 und 38 genutzt werden, um die Clips 14 und 16 voneinander zu unterscheiden. Hierfür dienen die Unterscheidungselemente 34 und 106 an den Applikationsinstrumenten 12 und 98, die mit den Endbereichen 20 und 38 derart zusammenwirken, dass in jedem der Applikationsinstrumente 12 und 98 nur jeweils ein Clip 14 beziehungsweise 16 aufnehmbar und applizierbar ist.

Auf die beschriebene Weise können Verwechslungen der Clips 14 und 16 bei chirurgischen Eingriffen vermieden werden. Dies ist insbesondere dann vorteilhaft, wenn die Clips 14 und 16 unterschiedliche Formen, insbesondere im Bereich der Verbindungsabschnitte 22 und 40, aufweisen sowie unterschiedliche Schließkräfte definieren.

Anders als beispielsweise bei farblichen Markierungen zum Zuordnen von Clips zu korrespondierenden Applikationsinstrumenten haben die vorgeschlagenen Ausführungsbeispiele von Applikationssystemen 10 den Vorteil, dass auch unter schlechten Sichtbedingungen eine Verwechslung zwischen Clips 14 beziehungsweise 16 einerseits und den Applikationsinstrumenten 12 und 98 andererseits, ausgeschlossen werden kann.

### Bezugszeichenliste

- 10: Applikationssystem
- 12: erstes Applikationsinstrument
- 14: erster medizinischer Clip
- 16: zweiter medizinischer Clip
- 18: erste Werkzeugelemente
- 20: erster proximaler Endbereich
- 22: erster Verbindungsabschnitt
- 24: erster Volumenbereich
- 26: erster Klemmarm
- 28: erste Endbereichsaufnahme
- 30: erste Verbindungsabschnittsaufnahme
- 32: erster Aufnahmeabschnitt
- 34: erstes Unterscheidungselement
- 36: zweiter Volumenbereich
- 38: zweiter Endbereich
- 40: zweite Verbindungsabschnitte
- 42: zweite Aufnahmeabschnitte
- 44: erste Betätigungseinrichtung
- 46: erste Schwenkachse
- 48: erstes vorspannendes Element
- 50: erste Schraubenfeder
- 52: zweites vorspannendes Element
- 54: zweite Schraubenfeder
- 56: zweiter Klemmarme
- 58: erster Schlussbereich
- 60: zweiter Schlussbereich
- 62: Durchsteckschluss
- 64: Durchsteckschluss
- 66: Schlussbereichsdurchbrechung
- 68: erste Clipebene
- 70: zweite Clipebene
- 72: erste Schraubenfederlängsachse
- 74: zweite Schraubenfederlängsachse
- 76: erste Schraubenfederebene
- 78: zweite Schraubenfederebene
- 80: erster Neigungswinkel
- 82: zweiter Neigungswinkel
- 84: erstes Kodierelement
- 86: erster Kodiervorsprung
- 88: Anlagefläche
- 90: Seite
- 92: Seite
- 94: Ende
- 96: erste Längsachse
- 98: zweites Applikationsinstrument
- 100: zweites Werkzeugelemente
- 102: zweite Endbereichsaufnahme
- 104: zweite Verbindungsabschnittsaufnahme
- 106: zweites Unterscheidungselement
- 108: zweite Betätigungseinrichtung
- 110: zweite Schwenkachse
- 112: zweites Kodierelement
- 114: zweiter Kodiervorsprung
- 116: Anlagefläche
- 118: Seite
- 120: Seite
- 122: zweite Längsachse
- 124: erster Volumenbereich
- 134: Unterscheidungselement
- 136: zweiter Volumenbereich
- 150: Wulst
- 152: Schlussplättchen
- 154: Wulstaussparung
- 160: Unterscheidungselementaufnahme

## Patentansprüche

1. Applikationssystem (10) für medizinische Clips, umfassend mindestens ein erstes Applikationsinstrument (12) zum Anlegen eines ersten Typs eines medizinischen Clips (14) und mindestens einen ersten medizinischen Clip (14) vom ersten Typ, wobei das erste Applikationsinstrument (12) zwei relativ zueinander bewegbare erste Werkzeugelemente (18) umfasst, wobei der mindestens eine erste medizinische Clip (14) vom ersten Typ einen ersten proximalen Endbereich (20) und zwei erste Verbindungsabschnitte (22) aufweist, welche sich distalseitig an den ersten, einen ersten Volumenbereich (24) definierenden proximalen Endbereich (20) anschließen und den ersten proximalen Endbereich (20) mit zwei relativ zueinander bewegbaren Klemmarmen (26) des mindestens einen ersten Clips (14) vom ersten Typ verbinden, wobei die zwei ersten Werkzeugelemente (18) zwischen sich eine in distaler Richtung geöffnete erste Endbereichsaufnahme (28) zum formschlüssigen oder im Wesentlichen formschlüssigen Aufnehmen des ersten proximalen Endbereichs (20) und jeweils eine erste Verbindungsabschnittsaufnahme (30) zum mindestens teilweisen Aufnehmen jeweils eines ersten Aufnahmeabschnitts (32) der ersten Verbindungsabschnitte (22) umfassen, wobei an mindestens einem der zwei ersten Werkzeugelemente (18) mindestens ein erstes Unterscheidungselement (34) derart angeordnet oder ausgebildet ist, dass es in einen zweiten Volumenbereich (36) eingreift, welcher von einem zweiten proximalen Endbereich (38) eines zweiten medizinischen Clips (16) von einem zweiten Typ definiert wird, wobei sich vom zweiten proximalen Endbereich (38) in distaler Richtung zwei zweite, jeweils einen zweiten Aufnahmeabschnitt (42) umfassende Verbindungsabschnitte (40) weg erstrecken, und wobei der erste Volumenbereich (24) und der zweite Volumenbereich (36) nur teilweise überlappen unter der Zwangsbedingung, dass die zweiten Aufnahmeabschnitte (42) in jeweils einer ersten Verbindungsabschnittsaufnahme (30) der zwei ersten Werkzeugelemente (18) aufgenommen sind, **dadurch gekennzeichnet, dass** das Applikationssystem (10) mindestens ein zweites Applikationsinstrument (98) zum Anlegen eines zweiten Typs eines medizinischen Clips (16) und mindestens einen zweiten medizinischen Clip (16) vom zweiten Typ umfasst, welcher zwei relativ zueinander bewegbare zweite Klemmarme (56), die sich distalseitig an die zwei zweiten Verbindungsabschnitte (40) anschließen, umfasst, dass das mindestens eine zweite Applikationsinstrument (98) zwei relativ zueinander bewegbare zweite Werkzeugelemente (100) umfasst, dass die zwei zweiten Werkzeugelemente (100) zwischen sich eine in distaler Richtung geöffnete zweite Endbereichsaufnahme (102) zum formschlüssigen oder im Wesentlichen formschlüssigen Aufnehmen des zweiten proximalen Endbereichs (38) und jeweils eine zweite Verbindungsabschnittsaufnahme (104) zum mindestens teilweisen Aufnehmen jeweils eines der zweiten Aufnahmeabschnitte (42) umfassen, dass an mindestens einem der zwei zweiten Werkzeugelemente (100) mindestens ein zweites Unterscheidungselement (106) derart angeordnet oder ausgebildet ist, dass es in den ersten Volumenbereich (24) eingreift unter der Zwangsbedingung, dass die ersten Aufnahmeabschnitte (32) in jeweils einer zweiten Verbindungsabschnittsaufnahme (104) der zwei zweiten Werkzeugelemente (100) aufgenommen sind.

2. Applikationssystem nach Anspruch 1, **dadurch gekennzeichnet, dass** das mindestens eine zweite Applikationsinstrument (98) eine zweite Betätigungseinrichtung (108) umfasst zum Bewegen, insbesondere zum Verschwenken, der zwei zweiten Werkzeugelemente (100) relativ zueinander von einer zweiten Aufnahmestellung, in welcher ein zum zweiten Applikationsinstrument (98) korrespondierender zweiter medizinischer Clip (16) vom zweiten Typ mit seinem zweiten Endbereich (38) in der zweiten Endbereichsaufnahme (102) aufnehmbar ist, in eine zweite Applikationsstellung, in welcher die zwei zweiten Werkzeugelemente (100) aufeinander zu und die zweiten Klemmarme (56) voneinander weg bewegt sind zum Applizieren des zweiten Clips (16) vom zweiten Typ, wobei insbesondere die zweiten Werkzeugelemente (100) des zweiten Applikationsinstruments (98) den zweiten proximalen Endbereich (38) in der zweiten Aufnahmestellung und in der zweiten Applikationsstellung teilweise umgreifen.

3. Applikationssystem nach einem der voranstehenden Ansprüche, **dadurch gekennzeichnet, dass** die zwei zweiten Werkzeugelemente (100) des mindestens einen zweiten Applikationsinstruments (98) relativ zueinander bewegbar sind, insbesondere um eine gemeinsame zweite Schwenkachse (110) relativ zueinander verschwenkbar sind.

4. Applikationssystem nach einem der voranstehenden Ansprüche, **dadurch gekennzeichnet, dass**
a) das mindestens eine erste Applikationsinstrument (12) eine erste Betätigungseinrichtung (44) umfasst zum Bewegen, insbesondere zum Verschwenken, der zwei ersten Werkzeugelemente (18) relativ zueinander von einer ersten Aufnahmestellung, in welcher ein zum ersten Applikationsinstrument (12) korrespondierender erster medizinischer Clip (14) vom ersten Typ mit seinem ersten Endbereich (20) in der ersten Endbereichsaufnahme (28) aufnehmbar ist, in eine erste Applikationsstellung, in welcher die zwei ersten Werkzeugelemente (18) aufeinander zu und die ersten Klemmarme (26) voneinander weg bewegt sind zum Applizieren des ersten Clips (14) vom ersten Typ,
wobei insbesondere die ersten Werkzeugelemente (18) des ersten Applikationsinstruments (12) den ersten proximalen Endbereich (20) in der ersten Aufnahmestellung und in der ersten Applikationsstellung teilweise umgreifen,
und/oder
b) die zwei ersten Werkzeugelemente (18) des mindestens einen ersten Applikationsinstruments (12) relativ zueinander bewegbar sind, insbesondere um eine gemeinsame erste Schwenkachse (46) relativ zueinander verschwenkbar sind,
und/oder
c) sich die ersten Aufnahmeabschnitte (32) direkt oder im Wesentlichen direkt an den ersten proximalen Endbereich (20) anschließen und/oder dass sich die zweiten Aufnahmeabschnitte (42) direkt oder im Wesentlichen direkt an den zweiten proximalen Endbereich (38).

5. Applikationssystem nach einem der voranstehenden Ansprüche, **dadurch gekennzeichnet, dass** der erste proximale Endbereich (20) ein erstes vorspannendes Element (48) umfasst und/oder dass der zweite proximale Endbereich (38) ein zweites vorspannendes Element (52) umfasst wobei insbesondere
a) das erste vorspannende Element (48) in Form einer ersten Schraubenfeder (50) mit mindestens einer vollständigen Windung ausgebildet ist und/oder dass das zweite vorspannende Element (52) in Form einer zweiten Schraubenfeder (54) mit mindestens einer vollständigen Windung ausgebildet ist
und/oder
b) das erste vorspannende Element (48) und/oder das zweite vorspannende Elemente (52) durch einen Kaltumformprozess ausgebildet sind.

6. Applikationssystem nach einem der voranstehenden Ansprüche, **dadurch gekennzeichnet, dass** die ersten Klemmarme (26) in einer ersten Grundstellung des mindestens einen ersten Clips (14) vom ersten Typ aneinander anliegen und entgegen der Wirkung des ersten vorspannenden Elements (48) von der ersten Grundstellung in eine erste Öffnungsstellung voneinander weg bewegbar sind und/oder dass die zweiten Klemmarme (56) in einer zweiten Grundstellung des mindestens einen zweiten Clips (16) vom zweiten Typ aneinander anliegen und entgegen der Wirkung des zweiten vorspannenden Elements (52) von der zweiten Grundstellung in eine zweite Öffnungsstellung voneinander weg bewegbar sind.

7. Applikationssystem nach einem der voranstehenden Ansprüche, **dadurch gekennzeichnet, dass**
a) sich die ersten Verbindungsabschnitte (22) in einem ersten Schlussbereich (58) des mindestens einen ersten Clips (14) vom ersten Typ kreuzen,
wobei insbesondere der erste Schlussbereich (58)
- distalseitig der ersten Aufnahmeabschnitte (32) angeordnet oder ausgebildet
und/oder
- in Form eines Durchsteckschlusses (62, 64) ausgebildet ist,
und/oder
b) sich die zweiten Verbindungsabschnitte (40) in einem zweiten Schlussbereich (60) des mindestens einen zweiten Clips (16) vom zweiten Typ kreuzen,
wobei insbesondere der zweite Schlussbereich (60)
- distalseitig der zweiten Aufnahmeabschnitte (42) angeordnet oder ausgebildet ist
und/oder
- in Form eines Durchsteckschlusses (62, 64) ausgebildet ist.

8. Applikationssystem nach einem der voranstehenden Ansprüche, **dadurch gekennzeichnet, dass** der erste proximale Endbereich (20) ein erstes vorspannendes Element (48) umfasst, dass der zweite proximale Endbereich (38) ein zweites vorspannendes Element (52) umfasst, dass das erste vorspannende Element (48) in Form einer ersten Schraubenfeder (50) mit mindestens einer vollständigen Windung ausgebildet ist, dass das zweite vorspannende Element (52) in Form einer zweiten Schraubenfeder (54) mit mindestens einer vollständigen Windung ausgebildet ist, dass der mindestens eine erste medizinische Clip (14) vom ersten Typ eine erste Clipebene (68) definiert, dass die erste Schraubenfeder (50) eine erste Schraubenfederlängsachse (72) definiert, dass die erste Schraubenfederlängsachse (72) senkrecht zu einer ersten Schraubenfederebene (76) verläuft und dass die erste Schraubenfederebene (76) relativ zur ersten Clipebene (68) um einen ersten Neigungswinkel (80) geneigt ist und/oder dass der mindestens eine zweite medizinische Clip (16) vom zweiten Typ eine zweite Clipebene (70) definiert, dass die zweite Schraubenfeder (54) eine zweite Schraubenfederlängsachse (74) definiert, dass die zweite Schraubenfederlängsachse (74) senkrecht zu einer zweiten Schraubenfederebene (78) verläuft und dass die zweite Schraubenfederebene (78) relativ zur zweiten Clipebene (70) um einen zweiten Neigungswinkel (82) geneigt ist.

9. Applikationssystem nach Anspruch 8, **dadurch gekennzeichnet, dass** die erste Schraubenfeder (50) im Uhrzeigersinn gewickelt ist und dass der erste Neigungswinkel (80) einen positiven Wert aufweist und/oder dass die zweite Schraubenfeder (54) im Gegenuhrzeigersinn gewickelt ist und dass der zweite Neigungswinkel (82) einen negativen Wert aufweist.

10. Applikationssystem nach Anspruch 8 oder 9, **dadurch gekennzeichnet, dass** der erste Neigungswinkel (80) einen Wert in einem Bereich zwischen 0° und etwa 40° aufweist, insbesondere einen Wert in einem Bereich zwischen 0° und etwa 20°, und/oder dass der zweite Neigungswinkel (82) einen Wert in einem Bereich zwischen 0° und etwa -40° aufweist, insbesondere einen Wert in einem Bereich zwischen 0° und etwa -20°.

11. Applikationssystem nach Anspruch 9 oder 10, **dadurch gekennzeichnet, dass**
a) das mindestens eine erste Applikationsinstrument (12) korrespondierend zu einem ersten medizinischen Clip (14) vom ersten Typ mit einer im Uhrzeigersinn gewickelten ersten Schraubenfeder (50) ausgebildet ist
und/oder
b) das mindestens eine zweite Applikationsinstrument (98) korrespondierend zu einem zweiten medizinischen Clip (16) vom zweiten Typ mit einer im Gegenuhrzeigersinn gewickelten zweiten Schraubenfeder (54) ausgebildet ist.

12. Applikationssystem nach einem der voranstehenden Ansprüche, **dadurch gekennzeichnet, dass** das mindestens eine erste Unterscheidungselement (34) in Form eines mit einem beliebigen medizinischen Clip zusammenwirkendes erstes Kodierelement (84) umfasst und dass in der ersten Endbereichsaufnahme (28) des ersten Applikationsinstruments (12) ausschließlich der zum ersten Kodierelement (84) korrespondierende erste medizinische Clip (14) vom ersten Typ formschlüssig oder im Wesentlichen formschlüssig aufnehmbar ist,
wobei insbesondere
a) das mindestens eine erste Kodierelement (84) in Form eines ersten Kodiervorsprungs (86) ausgebildet ist, welcher die erste Endbereichsaufnahme (28) mindestens abschnittsweise seitlich begrenzt
und/oder
b) das erste Applikationsinstrument (12) zwei mit einem beliebigen medizinischen Clip zusammenwirkende erste Kodierelemente (84) umfasst und dass die ersten Kodierelemente (84) seitliche Anlageflächen (88) für voneinander weg weisende Seiten (90, 92) ausschließlich für den in der ersten Endbereichsaufnahme (28) aufgenommenen ersten Endbereich (20) bilden.

13. Applikationssystem nach einem der voranstehenden Ansprüche, **dadurch gekennzeichnet, dass** das mindestens eine zweite Unterscheidungselement (106) in Form eines mit einem beliebigen medizinischen Clip zusammenwirkendes zweites Kodierelement (112) umfasst und dass in der zweiten Endbereichsaufnahme (102) des zweiten Applikationsinstruments (98) ausschließlich der zum zweiten Kodierelement (112) korrespondierende zweite medizinische Clip (16) vom zweiten Typ formschlüssig oder im Wesentlichen formschlüssig aufnehmbar ist,
wobei insbesondere
a) das mindestens eine zweite Kodierelement (112) in Form eines zweiten Kodiervorsprungs (114) ausgebildet ist, welcher die zweite Endbereichsaufnahme (102) mindestens abschnittsweise seitlich begrenzt
und/oder
b) das zweite Applikationsinstrument (98) zwei mit einem beliebigen medizinischen Clip zusammenwirkende zweite Kodierelemente (112) umfasst und dass die zweiten Kodierelemente seitliche Anlageflächen (116) für voneinander weg weisende Seiten (118, 120) ausschließlich für den in der zweiten Endbereichsaufnahme (102) aufgenommenen zweiten Endbereich (38) bilden.

14. Applikationssystem nach Anspruch 12 oder 13, **dadurch gekennzeichnet, dass**
a) an jedem ersten Werkzeugelement (18) des ersten Applikationsinstruments (12) jeweils eines der beiden ersten Kodierelemente (84) angeordnet oder ausgebildet ist und/oder dass an jedem zweiten Werkzeugelement (100) des zweiten Applikationsinstruments (98) jeweils eines der beiden zweiten Kodierelemente (112) angeordnet oder ausgebildet ist
und/oder
b) das mindestens eine erste Kodierelement (84) am einen der zwei ersten Werkzeugelemente (18) in distaler Richtung über ein proximales Ende (94) des die erste Endbereichsaufnahme (28) begrenzenden anderen ersten Werkzeugelements (18) vorstehend ausgebildet ist und/oder dass das mindestens eine zweite Kodierelement (112) am einen der zwei zweiten Werkzeugelemente (100) in distaler Richtung über ein proximales Ende des die zweite Endbereichsaufnahme (102) begrenzenden anderen zweiten Werkzeugelements (100) vorstehend ausgebildet ist.

15. Applikationssystem nach einem der voranstehenden Ansprüche, **dadurch gekennzeichnet, dass**
a) die zwei ersten Werkzeugelemente (18) im Bereich der ersten Endbereichsaufnahme (28) durch Drehung um 180° bezogen auf eine erste Längsachse (96) der ersten Endbereichsaufnahme (28) ineinander überführbar sind und/oder dass die zwei zweiten Werkzeugelemente (100) im Bereich der zweiten Endbereichsaufnahme (102) durch Drehung um 180° bezogen auf eine zweite Längsachse (122) der zweiten Endbereichsaufnahme (102) ineinander überführbar sind
und/oder
b) der mindestens eine erste medizinische Clip (14) vom ersten Typ und/oder der mindestens eine zweite medizinische Clip (16) vom zweiten Typ in Form von Aneurysmenclips ausgebildet sind.

## Claims

1. Application system (10) for medical clips, comprising at least one first application instrument (12) for applying a first type of a medical clip (14) and at least one first medical clip (14) of the first type, wherein the first application instrument (12) comprises two first tool elements (18) that are movable relative to one another, wherein the at least one first medical clip (14) of the first type has a first proximal end region (20) and two first connecting portions (22), which distally adjoin the first proximal end region (20) defining a first volume region (24) and connect the first proximal end region (20) to two clamping arms (26) of the at least one first clip (14) of the first type that are movable relative to one another, wherein the two first tool elements (18) comprise between them a first end region receptacle (28) that is open in the distal direction for accommodating the first proximal end region (20) in a positive-locking or substantially positive-locking manner and each comprise a first connecting portion receptacle (30) for at least partially accommodating a respective first accommodation portion (32) of the first connecting portions (22), wherein at least one first distinguishing element (34) is arranged or formed on at least one of the two first tool elements (18) in such a way that it engages into a second volume region (36), which is defined by a second proximal end region (38) of a second medical clip (16) of a second type, wherein two second connecting portions (40), each comprising a second accommodation portion (42), extend away from the second proximal end region (38) in the distal direction, and wherein the first volume region (24) and the second volume region (36) only partially overlap under the constraint that the second accommodation portions (42) are each accommodated in a first respective connecting portion receptacle (30) of the two first tool elements (18), **characterized in that** the application system (10) comprises at least one second application instrument (98) for applying a second type of a medical clip (16) and at least one second medical clip (16) of the second type, which comprises two second clamping arms (56) that are movable relative to one another and that distally adjoin the two second connecting portions (40), **in that** the at least one second application instrument (98) comprises two second tool elements (100) that are movable relative to one another, **in that** the two second tool elements (100) comprise between them an end region receptacle (102) open in the distal direction for accommodating the second proximal end region (38) in a positive-locking or substantially positive-locking manner and each comprise a second connecting portion receptacle (104) for at least partially accommodating a respective one of the second accommodation portions (42), **in that** at least one second distinguishing element (106) is arranged or formed on at least one of the two second tool elements (100) in such a way that it engages into the first volume region (24) under the constraint that the first accommodation portions (32) are each accommodated in a respective second connecting portion receptacle (104) of the two second tool elements (100).

2. Application system in accordance with Claim 1**, characterized in that** the at least one second application instrument (98) comprises a second actuating device (108) for moving, in particular for pivoting, the two second tool elements (100) relative to one another from a second accommodation position, in which a second medical clip (16) of the second type corresponding to the second application instrument (98) can be accommodated with its second end region (38) in the second end region receptacle (102), into a second application position, in which the two second tool elements (100) are moved toward one another and the second clamping arms (56) are moved away from one other for applying the second clip (16) of the second type,
wherein, in particular, the second tool elements (100) of the second application instrument (98) partially engage around the second proximal end region (38) in the second accommodation position and in the second application position.

3. Application system in accordance with any one of the preceding Claims, **characterized in that** the two second tool elements (100) of the at least one second application instrument (98) are movable relative to one another, in particular are pivotable relative to one another about a common second pivot axis (110).

4. Application system in accordance with any one of the preceding Claims, **characterized in that**
a) the at least one first application instrument (12) comprises a first actuating device (44) for moving, in particular for pivoting, the two first tool elements (18) relative to one another from a first accommodation position, in which a first medical clip (14) of the first type corresponding to the first application instrument (12) can be accommodated with its first end region (20) in the first end region receptacle (28), into a first application position, in which the two first tool elements (18) are moved toward one another and the first clamping arms (26) are moved away from one other for applying the first clip (14) of the first type,
wherein, in particular, the first tool elements (18) of the first application instrument (12) partially engage around the first proximal end region (20) in the first accommodation position and in the first application position,
and/or
b) the two first tool elements (18) of the at least one first application instrument (12) are movable relative to one another, in particular are pivotable relative to one another about a common first pivot axis (46)
and/or
c) the first accommodation portions (32) directly or substantially directly adjoin the first proximal end region (20) and/or **in that** the second accommodation portions (42) directly or substantially directly adjoin the second proximal end region (38).

5. Application system in accordance with any one of the preceding Claims, **characterized in that** the first proximal end region (20) comprises a first biasing element (48) and/or **in that** the second proximal end region (38) comprises a second biasing element (52),
wherein, in particular,
a) the first biasing element (48) is configured in the form of a first coil spring (50) with at least one complete winding and/or **in that** the second biasing element (52) is configured in the form of a second coil spring (54) with at least one complete winding
and/or
b) the first biasing element (48) and/or the second biasing element (52) are made by a cold forming process.

6. Application system in accordance with any one of the preceding Claims, **characterized in that** the first clamping arms (26) abut against one another in a first basic position of the at least one first clip (14) of the first type and are movable away from one another against the action of the first biasing element (48) from the first basic position into a first opening position and/or **in that** the second clamping arms (56) abut against one another in a second basic position of the at least one second clip (16) of the second type and are movable away from one another against the action of the second biasing element (52) from the second basic position into a second opening position.

7. Application system in accordance with any one of the preceding Claims, **characterized in that**
a) the first connecting portions (22) intersect in a first connection region (58) of the at least one first clip (14) of the first type, wherein, in particular, the first connection region (58)
- is arranged or formed on the distal side of the first accommodation portions (32)
and/or
- is configured in the form of a box lock (62, 64),
and/or
b) the second connecting portions (40) intersect in a second connection region (60) of the at least one second clip (16) of the second type,
wherein, in particular, the second connection region (60)
- is arranged or formed on the distal side of the second accommodation portions (42)
and/or
- is configured in the form of a box lock (62, 64).

8. Application system in accordance with any one of the preceding Claims, **characterized in that** the first proximal end region (20) comprises a first biasing element (48), **in that** the second proximal end region (38) comprises a second biasing element (52), **in that** the first biasing element (48) is configured in the form of a first coil spring (50) with at least one complete winding, **in that** the second biasing element (52) is configured in the form of a second coil spring (54) with at least one complete winding, **in that** the at least one first medical clip (14) of the first type defines a first clip plane (68), **in that** the first coil spring (50) defines a first coil spring longitudinal axis (72), **in that** the first coil spring longitudinal axis (72) extends perpendicularly to a first coil spring plane (76), and **in that** the first coil spring plane (76) is inclined relative to the first clip plane (68) by a first angle of inclination (80) and/or **in that** the at least one second medical clip (16) of the second type defines a second clip plane (70), **in that** the second coil spring (54) defines a second coil spring longitudinal axis (74), **in that** the second coil spring longitudinal axis (74) extends perpendicularly to a second coil spring plane (78), and **in that** the second coil spring plane (78) is inclined relative to the second clip plane (70) by a second angle of inclination (82).

9. Application system in accordance with Claim 8, **characterized in that** the first coil spring (50) is wound clockwise and **in that** the first angle of inclination (80) has a positive value and/or **in that** the second coil spring (54) is wound counterclockwise and **in that** the second angle of inclination (82) has a negative value.

10. Application system in accordance with Claim 8 or 9, **characterized in that** the first angle of inclination (80) has a value in a range between 0° and about 40°, in particular a value in a range between 0° and about 20°, and/or **in that** the second angle of inclination (82) has a value in a range between 0° and about -40°, in particular a value in a range between 0° and about -20°.

11. Application system in accordance with Claim 9 or 10, **characterized in that**
a) the at least one first application instrument (12) is configured corresponding to a first medical clip (14) of the first type with a first coil spring (50) wound clockwise
and/or
b) the at least one second application instrument (98) is configured corresponding to a second medical clip (16) of the second type with a second coil spring (54) wound counter-clockwise.

12. Application system in accordance with any one of the preceding Claims, **characterized in that** the at least one first distinguishing element (34) is configured in the form of a first coding element (84) that cooperates with any medical clip and **in that** exclusively the first medical clip (14) of the first type corresponding to the first coding element (84) can be accommodated in the first end region receptacle (28) of the first application instrument (12) in a positive-locking or substantially positive-locking manner,
wherein, in particular,
a) the at least one first coding element (84) is configured in the form of a first coding projection (86), which laterally delimits the first end region receptacle (28) at least in sections,
and/or
b) the first application instrument (12) comprises two first coding elements (84) that cooperate with any medical clip, and **in that** the first coding elements (84) form lateral abutment surfaces (88) for sides (90, 92) pointing away from one another exclusively for the first end region (20) accommodated in the first end region receptacle (28).

13. Application system in accordance with any one of the preceding Claims, **characterized in that** the at least one second distinguishing element (106) is configured in the form of a second coding element (112) that cooperates with any medical clip and **in that** exclusively the second medical clip (16) of the second type corresponding to the second coding element (112) can be accommodated in the second end region receptacle (102) of the second application instrument (98) in a positive-locking or substantially positive-locking manner,
wherein, in particular,
a) the at least one second coding element (112) is configured in the form of a second coding projection (114), which laterally delimits the second end region receptacle (102) at least in sections,
and/or
b) the second application instrument (98) comprises two second coding elements (112) that cooperate with any medical clip, and **in that** the second coding elements form lateral abutment surfaces (116) for sides (118, 120) pointing away from one another exclusively for the second end region (38) accommodated in the second end region receptacle (102).

14. Application system in accordance with Claim 12 or 13, **characterized in that**
a) a respective one of the two first coding elements (84) is arranged or formed on each first tool element (18) of the first application instrument (12) and/or **in that** a respective one of the two second coding elements (112) is arranged or formed on each second tool element (100) of the second application instrument (98),
and/or
b) the at least one first coding element (84) is formed on the one of the two first tool elements (18) projecting in the distal direction beyond a proximal end (94) of the other first tool element (18) delimiting the first end region receptacle (28) and/or **in that** the at least one second coding element (112) is formed on the one of the two second tool elements (100) projecting in the distal direction beyond a proximal end of the other second tool element (100) delimiting the second end region receptacle (102).

15. Application system in accordance with any one of the preceding Claims, **characterized in that**
a) the two first tool elements (18) are convertible into one another in the region of the first end region receptacle (28) by rotation by 180° relative to a first longitudinal axis (96) of the first end region receptacle (28) and/or **in that** the two second tool elements (100) are convertible into one another in the region of the second end region receptacle (102) by rotation by 180° relative to a second longitudinal axis (122) of the second end region receptacle (102) and/or
b) the at least one first medical clip (14) of the first type and/or the at least one second medical clip (16) of the second type are configured in the form of aneurysm clips.

## Revendications

1. Système d'application (10) pour clips médicaux, comprenant au moins un premier instrument d'application (12) pour appliquer un premier type de clip médical (14) et au moins un premier clip médical (14) du premier type, le premier instrument d'application (12) comprenant deux premiers éléments d'outil (18) mobiles l'un par rapport à l'autre, l'au moins un premier clip médical (14) du premier type présentant une première zone d'extrémité proximale (20) et deux premières sections de liaison (22) qui se raccordent distalement à la première zone d'extrémité proximale (20) définissant une première zone de volume (24) et relient la première zone d'extrémité proximale (20) avec deux bras de serrage (26) mobiles l'un par rapport à l'autre de l'au moins un premier clip (14) du premier type, les deux premiers éléments d'outil (18) comprenant entre eux un premier logement de zone d'extrémité (28) ouvert dans la direction distale pour loger par complémentarité de forme ou essentiellement par complémentarité de forme la première zone d'extrémité proximale (20) et, respectivement, un premier logement de section de liaison (30) pour loger au moins partiellement, respectivement, une première section de logement (32) des premières sections de liaison (22), au moins un premier élément de différenciation (34) étant agencé ou réalisé sur au moins l'un des deux premiers éléments d'outil (18) de telle sorte qu'il s'engage dans une deuxième zone de volume (36) qui est définie par une deuxième zone d'extrémité proximale (38) d'un deuxième clip médical (16) d'un deuxième type, deux deuxièmes sections de liaison (40) s'étendant dans la direction distale à partir de la deuxième zone d'extrémité proximale (38) et comprenant chacune une deuxième section de logement (42), et la première zone volumique (24) et la deuxième zone volumique (36) ne se chevauchant que partiellement sous la condition contraignante que les deuxièmes sections de logement (42) sont logées chacune dans un premier logement de section de liaison (30) des deux premiers éléments d'outil (18), **caractérisé en ce que** le système d'application (10) comprend au moins un deuxième instrument d'application (98) destiné à appliquer un deuxième type de clip médical (16) et comprenant au moins un deuxième clip médical (16) du deuxième type, qui comprend deux deuxièmes bras de serrage mobiles l'un par rapport à l'autre (56) qui se raccordent distalement aux deux deuxièmes sections de liaison (40), **en ce que** l'au moins un deuxième instrument d'application (98) comprend deux deuxièmes éléments d'outil (100) mobiles l'un par rapport à l'autre, **en ce que** les deux deuxièmes éléments d'outil (100) comprennent entre eux un deuxième logement de zone d'extrémité (102) ouvert dans la direction distale pour loger par complémentarité de forme ou essentiellement par complémentarité de forme la deuxième zone d'extrémité proximale (38) et, respectivement, un deuxième logement de section de liaison (104) pour loger au moins partiellement, respectivement, l'une des deux deuxièmes sections de logement (42), **en ce qu'**au moins un deuxième élément de différenciation (106) sur au moins un des deux deuxièmes éléments d'outil (100) est agencé ou réalisé de telle sorte qu'il s'engage dans la première zone volumique (24) sous la condition contraignante que les premières sections de logement (32) sont logées respectivement dans un deuxième logement de section de liaison (104) des deux deuxièmes éléments d'outil (100).

2. Système d'application selon la revendication 1, **caractérisé en ce que** l'au moins un deuxième instrument d'application (98) comprend un deuxième dispositif d'actionnement (108) pour déplacer, en particulier pour faire pivoter, les deux deuxièmes éléments d'outil (100) l'un par rapport à l'autre depuis une deuxième position de logement, dans laquelle un deuxième clip médical (16) de deuxième type correspondant au deuxième instrument d'application (98) peut être logé avec sa deuxième zone d'extrémité (38) dans le deuxième logement de zone d'extrémité (102), vers une deuxième position d'application, dans laquelle les deux deuxièmes éléments d'outil (100) sont déplacés l'un vers l'autre et les deuxièmes bras de serrage (56) sont éloignés l'un de l'autre pour appliquer le deuxième clip (16) du deuxième type,
les deuxièmes éléments d'outil (100) du deuxième instrument d'application (98), en particulier, entourant partiellement la deuxième zone d'extrémité proximale (38) dans la deuxième position de logement et dans la deuxième position d'application.

3. Système d'application selon l'une des revendications précédentes, **caractérisé en ce que** les deux deuxièmes éléments d'outil (100) de l'au moins un deuxième instrument d'application (98) sont mobiles l'un par rapport à l'autre, en particulier peuvent pivoter l'un par rapport à l'autre autour d'un deuxième axe de pivotement commun (110).

4. Système d'application selon l'une des revendications précédentes, **caractérisé en ce que**
a) l'au moins un premier instrument d'application (12) comprend un premier dispositif d'actionnement (44) pour déplacer, en particulier pour faire pivoter, les deux premiers éléments d'outil (18) l'un par rapport à l'autre à partir d'une première position de logement, dans laquelle un premier clip médical (14) d'un premier type, correspondant au premier instrument d'application (12), peut être logé avec sa première zone d'extrémité (20) dans le premier logement de zone d'extrémité (28), dans une première position d'application dans laquelle les deux premiers éléments d'outil (18) sont déplacés l'un vers l'autre et les premiers bras de serrage (26) sont éloignés l'un de l'autre pour appliquer le premier clip (14) du premier type,
les premiers éléments d'outil (18) du premier instrument d'application (12), en particulier, entourant partiellement la première zone d'extrémité proximale (20) dans la première position de logement et dans la première position d'application,
et/ou
b) les deux premiers éléments d'outil (18) de l'au moins un premier instrument d'application (12) étant mobiles l'un par rapport à l'autre, en particulier pouvant pivoter l'un par rapport à l'autre autour d'un premier axe de pivotement commun (46),
et/ou
c) les premières sections de logement (32) étant directement ou essentiellement directement reliées à la première zone d'extrémité proximale (20) et/ou les deuxièmes sections de logement (42) étant directement ou essentiellement directement reliées à la deuxième zone d'extrémité proximale (38).

5. Système d'application selon l'une des revendications précédentes, **caractérisé en ce que** la première zone d'extrémité proximale (20) comprend un premier élément de précontrainte (48) et/ou **en ce que** la deuxième zone d'extrémité proximale (38) comprend un deuxième élément de précontrainte (52),
dans lequel, en particulier,
a) le premier élément de précontrainte (48) est réalisé sous la forme d'un premier ressort hélicoïdal (50) avec au moins un tour complet et/ou que le deuxième élément de précontrainte (52) est réalisé sous la forme d'un deuxième ressort hélicoïdal (54) avec au moins un tour complet
et/ou
b) le premier élément de précontrainte (48) et/ou le deuxième élément de précontrainte (52) sont réalisés par un processus de formage à froid.

6. Système d'application selon l'une des revendications précédentes, **caractérisé en ce que** les premiers bras de serrage (26) sont en contact les uns avec les autres dans une première position de base de l'au moins un premier clip (14) du premier type et peuvent être éloignés l'un de l'autre à l'encontre de l'action du premier élément de précontrainte (48) de la première position de base vers une première position d'ouverture et/ou **en ce que** les deuxièmes bras de serrage (56) reposent les uns contre les autres dans une deuxième position de base de l'au moins un deuxième clip (16) du deuxième type et peuvent s'éloigner l'un de l'autre à l'encontre de l'action du deuxième élément de précontrainte (52) de la deuxième position de base vers une deuxième position d'ouverture.

7. Système d'application selon l'une des revendications précédentes, **caractérisé en ce que**
a) les premières sections de liaison (22) se croisent dans une première zone de fermeture (58) de l'au moins un premier clip (14) du premier type,
la première zone de fermeture (58), en particulier,
- étant agencée ou réalisée du côté distal des premières sections de logement (32)
et/ou
- étant réalisée sous la forme d'une fermeture enfichable (62, 64),
et/ou
b) les deuxièmes sections de liaison (40) se croisant dans une deuxième zone de fermeture (60) de l'au moins une deuxième clip (16) du deuxième type,
la deuxième zone de fermeture (60), en particulier,
- étant agencée ou réalisée du côté distal des deuxièmes sections de logement (42)
et/ou
- étant réalisée sous la forme d'une fermeture enfichable (62, 64).

8. Système d'application selon l'une des revendications précédentes, **caractérisé en ce que** la première zone d'extrémité proximale (20) comprend un premier élément de précontrainte (48), **en ce que** la deuxième zone d'extrémité proximale (38) comprend un deuxième élément de précontrainte (52), **en ce que** le premier élément de précontrainte (48) est réalisé sous la forme d'un premier ressort hélicoïdal (50) avec au moins un tour complet, **en ce que** le deuxième élément de précontrainte (52) est réalisé sous la forme d'un deuxième ressort hélicoïdal (54) avec au moins un tour complet, **en ce que** l'au moins un premier clip médical (14) du premier type définit un premier plan de clip (68), **en ce que** le premier ressort hélicoïdal (50) définit un premier axe longitudinal de ressort hélicoïdal (72), **en ce que** le premier axe longitudinal de ressort hélicoïdal (72) s'étend perpendiculairement à un premier plan de ressort hélicoïdal (76) et **en ce que** le premier plan de ressort hélicoïdal (76) est incliné par rapport au premier plan de clip (68) selon un premier angle d'inclinaison (80) et/ou **en ce que** l'au moins un deuxième clip médical (16) du deuxième type définit un deuxième plan de clip (70), **en ce que** le deuxième ressort hélicoïdal (54) définit un deuxième axe longitudinal de ressort hélicoïdal (74), **en ce que** le deuxième axe longitudinal de ressort hélicoïdal (74) s'étend perpendiculairement à un deuxième plan de ressort hélicoïdal (78) et **en ce que** le deuxième plan de ressort hélicoïdal (78) est incliné par rapport au deuxième plan de clip (70) selon un deuxième angle d'inclinaison (82).

9. Système d'application selon la revendication 8, **caractérisé en ce que** le premier ressort hélicoïdal (50) est enroulé dans le sens des aiguilles d'une montre et **en ce que** le premier angle d'inclinaison (80) présente une valeur positive et/ou **en ce que** le deuxième ressort hélicoïdal (54) est enroulé dans le sens inverse des aiguilles d'une montre et **en ce que** le deuxième angle d'inclinaison (82) présente une valeur négative.

10. Système d'application selon la revendication 8 ou 9, **caractérisé en ce que** le premier angle d'inclinaison (80) présente une valeur comprise entre 0° et environ 40°, en particulier une valeur comprise entre 0° et environ 20°, et/ou **en ce que** le deuxième angle d'inclinaison (82) présente une valeur comprise entre 0° et environ -40°, en particulier une valeur comprise entre 0° et environ -20°.

11. Système d'application selon la revendication 9 ou 10, **caractérisé en ce que**
a) l'au moins un premier instrument d'application (12) est réalisé correspondant à un premier clip médical (14) du premier type avec un premier ressort hélicoïdal (50) enroulé dans le sens des aiguilles d'une montre
et/ou
b) l'au moins un deuxième instrument d'application (98) est réalisé correspondant à un deuxième clip médical (16) du deuxième type avec un deuxième ressort hélicoïdal (54) enroulé dans le sens inverse des aiguilles d'une montre.

12. Système d'application selon l'une des revendications précédentes, **caractérisé en ce que** l'au moins un premier élément de différenciation (34) comprend un premier élément de codage (84) coopérant avec n'importe quelle clip médical et **en ce que**, dans le premier logement de zone d'extrémité (28) du premier instrument d'application (12), seul le premier clip médical (14) du premier type correspondant au premier élément de codage (84) peut être logé par complémentarité de forme ou essentiellement par complémentarité de forme,
où en particulier,
a) l'au moins un premier élément de codage (84) est réalisé sous la forme d'une première saillie de codage (86) qui délimite latéralement le premier logement de zone d'extrémité (28) au moins par sections,
et/ou
b) le premier instrument d'application (12) comprend deux premiers éléments de codage (84) coopérant avec n'importe quel clip médical et où les premiers éléments de codage (84) forment des surfaces d'appui latérales (88) pour des côtés (90, 92) orientés à l'opposé l'un de l'autre, exclusivement pour la première zone d'extrémité (20) logée dans le premier logement de zone d'extrémité (28).

13. Système d'application selon l'une des revendications précédentes, **caractérisé en ce que** l'au moins un deuxième élément de différenciation (106) comprend un deuxième élément de codage (112) coopérant avec n'importe quel clip médical et **en ce que**, dans le deuxième logement de zone d'extrémité (102) du deuxième instrument d'application (98), seul le deuxième clip médical (16) de deuxième type correspondant au deuxième élément de codage (112) peut être logé par complémentarité de forme ou essentiellement par complémentarité de forme,
où, en particulier,
a) l'au moins un deuxième élément de codage (112) est réalisé sous la forme d'une deuxième saillie de codage (114) qui délimite latéralement le deuxième logement de zone d'extrémité (102) au moins par sections,
et/ou
b) le deuxième instrument d'application (98) comprend deux deuxièmes éléments de codage (112) coopérant avec n'importe quel clip médical et **en ce que** les deuxièmes éléments de codage forment des surfaces d'appui latérales (116) pour des côtés (118, 120) orientés à l'opposé l'un de l'autre exclusivement pour la deuxième zone d'extrémité (38) logée dans le deuxième logement de zone d'extrémité (102).

14. Système d'application selon la revendication 12 ou 13, **caractérisé en ce que**
a) l'un des deux premiers éléments de codage (84) est agencé ou réalisé sur chaque premier élément d'outil (18) du premier instrument d'application (12) et/ou **en ce que** l'un des deux deuxièmes éléments de codage (112) est agencé ou réalisé sur chaque deuxième élément d'outil (100) du deuxième instrument d'application (98),
et/ou
b) l'au moins un premier élément de codage (84) sur l'un des deux premiers éléments d'outil (18) est formé en saillie dans la direction distale au-delà d'une extrémité proximale (94) de l'autre premier élément d'outil (18) délimitant le premier logement de zone d'extrémité (28) et/ou **en ce que** l'au moins un deuxième élément de codage (112) est réalisé sur l'un des deux deuxièmes éléments d'outil (100) dans la direction distale au-delà d'une extrémité proximale de l'autre deuxième élément d'outil (100) délimitant le deuxième logement de zone d'extrémité (102).

15. Système d'application selon l'une des revendications précédentes, **caractérisé en ce que**
a) les deux premiers éléments d'outil (18) peuvent être transférés l'un dans l'autre dans la zone du premier logement de zone d'extrémité (28) par une rotation de 180° par rapport à un premier axe longitudinal (96) du premier logement de zone d'extrémité (28) et/ou **en ce que** les deux deuxièmes éléments d'outil (100) peuvent être transférés l'un dans l'autre dans la zone du deuxième logement de zone d'extrémité (102) par une rotation de 180° par rapport à un deuxième axe longitudinal (122) du deuxième logement de zone d'extrémité (102)
et/ou
b) l'au moins un premier clip médical (14) du premier type et/ou l'au moins un deuxième clip médical (16) du deuxième type sont réalisés sous la forme de clips pour anévrisme.
